# EUROPEAN PATENT SPECIFICATION

(11) **EP 3 524 314 B1**
(45) Date of publication and mention of the grant of the patent: **29.05.2024**
(21) Application number: 19166777.3
(22) Date of filing: 21.08.2013
(51) Int. Cl.: A61M 37/00, A61M 39/02, A61B 90/00, A61B 90/94

(54) **IMPLANTABLE ACCESS PORT INCLUDING A SANDWICHED RADIOPAQUE INSERT**
IMPLANTIERBARE EINLASSÖFFNUNG MIT EINEM GESCHICHTETEN STRAHLUNGSUNDURCHLÄSSIGEN EINSATZ
PORT D'ACCÈS IMPLANTABLE COMPRENANT UN INSERT RADIO-OPAQUE EN SANDWICH

(30) Priority: 21.08.2012 US 201261691725 P; 25.02.2013 US 201313776517
(43) Date of publication of application: 14.08.2019
(62) Divisional of application: 13830592.5
(73) Proprietor: C. R. Bard, Inc., Franklin Lakes, NJ 07417 (US)
(72) Inventor: Wiley, Martha, Salt Lake City, UT 84108 (US); Eliasen, Kenneth A., Cottonwood Heights, UT 84122 (US); Hibdon, Dwight, Park City, UT 84098 (US); McKinnon, Melissa A., West Jordan, UT 84084 (US); Powers, Kelly B., North Salt Lake, UT 84054 (US); Cise, David M., Herriman, UT 84096 (US); Maniar, Ketan K., Mahwah, NJ 07430 (US)
(74) Representative: Hoffmann Eitle

(56) References cited:
- WO-A2-2011/046604
- US-A1- 2010 063 451
- US-A1- 2010 268 165
- US-A1- 2011 288 503

## Description

### Related Applications

This application claims the benefit of U.S. Provisional Application No. 61/691,725, filed August 21, 2012, and titled "Implantable Access Port Including Sandwiched Radiopaque Insert." This application is also a continuation-in part of U.S. Patent Application No. 13/776,517, filed February 25, 2013, which is a division of U.S. Patent Application No. 13/250,909, filed September 30, 2011, now U.S. Patent No. 8,382,724, which is a division of U.S. Patent Application No. 12/796,133, filed June 8. 2010, now U.S. Patent No. 8,029,482, which is a continuation-in-part of U.S. Patent Application No. 12/610,084, filed October 30, 2009, now U.S. Patent No. 8,202,259, which claims the benefit of U.S. Provisional Application No. 61/110.507, filed October 31, 2008, and titled "Radiopaque and Radiographically Discernible Indicators for an Implantable Port," and which is a continuation-in-part of U.S. Patent Application No. 12/420,028, filed April 7, 2009, now U.S. Patent No. 7,947,022, which is a continuation-in-part of the United States Patent Application No. 11/368,954, filed March 6, 2006, now U.S. Patent No. 7,785,302, which claims the benefit of U.S. Provisional Application No. 60/658,518, filed March 4, 2005, and titled "Access Port Identification System."

### Background

US 2010/0268165 A1 discloses an access port for subcutaneous implantation. Such an access port may comprise a body for capturing a septum for repeatedly inserting a needle therethrough into a cavity defined within the body. Further, the access port may include at least one feature structured and configured for identification of the access port subsequent to subcutaneous implantation. Methods of identifying a subcutaneously implanted access port are also disclosed. For example, a subcutaneously implanted access port may be provided and at least one feature of the subcutaneously implanted access port may be perceived. Further, the subcutaneously implanted access port may be identified in response to perceiving the at least one feature. In one embodiment, an identification feature is included on a molded insert that is incorporated into the access port so as to be visible after implantation via x-ray imaging technology.

### Summary

In a first aspect of the present invention, there is provided a method according to claim 1.

The dependent claims refer to preferred embodiments.

### Brief Description of the Drawings

FIG. 1A shows a perspective view of an embodiment of an access port according to the instant disclosure;
FIG. 1 B shows a schematic side cross-sectional view the access port shown in FIG. 1A;
FIG. 2 show's a perspective view of an embodiment of an access port according to the instant disclosure;
FIG. 3 shows a perspective view of an access port according to the instant disclosure;
FIG. 4 shows a perspective view of an access port according to the instant disclosure;
FIG. 5 shows a perspective view of an access port according to the instant disclosure;
FIG. 6A shows a perspective view of an access port according to the instant disclosure;
FIG. 6B shows a side view of the access port shown in FIG. 6A;
FIG. 7 shows a perspective view of an access port according to the instant disclosure;
FIG. 8 shows a simplified perspective view of a cap for forming an access port according to the instant disclosure;
FIG. 9 shows a simplified perspective view of a cap for forming an access port according to the instant disclosure;
FIG. 10 shows a simplified perspective view of a cap for forming an access port according to the instant disclosure;
FIG. 11 shows a simplified perspective view of a cap for forming an access port according to the instant disclosure;
FIG. 12 shows a simplified perspective view of a cap for forming an access port according to the instant disclosure;
FIG. 13 shows a simplified perspective view of a cap for forming an access port according to the instant disclosure;
FIG. 14 shows a simplified perspective view of a cap for forming an access port according to the instant disclosure;
FIG. 15A shows a perspective view of an embodiment of an access port according to the instant disclosure;
FIG. 15B shows a top elevation view of the access port shown in FIG. 15A;
FIG. 16 shows a perspective view of an access port according to the instant disclosure;
FIG. 17 shows a perspective view of an access port according to the instant disclosure;
FIG. 18 shows a perspective view of an access port according to the instant disclosure;
FIG. 19 shows a perspective view of an access port according to the instant disclosure;
FIG. 20 shows a perspective view of an access port according to the instant disclosure;
FIG. 21 shows a perspective view of an access port according to the instant disclosure;
FIG. 22 shows a perspective view of another embodiment of an access port according to the instant disclosure;
FIG. 23 shows a top elevation view of the assembled access port shown in FIG. 22;
FIG. 24 shows a simplified representation of a transverse cross section of the access port shown in FIGS. 22 and 23;

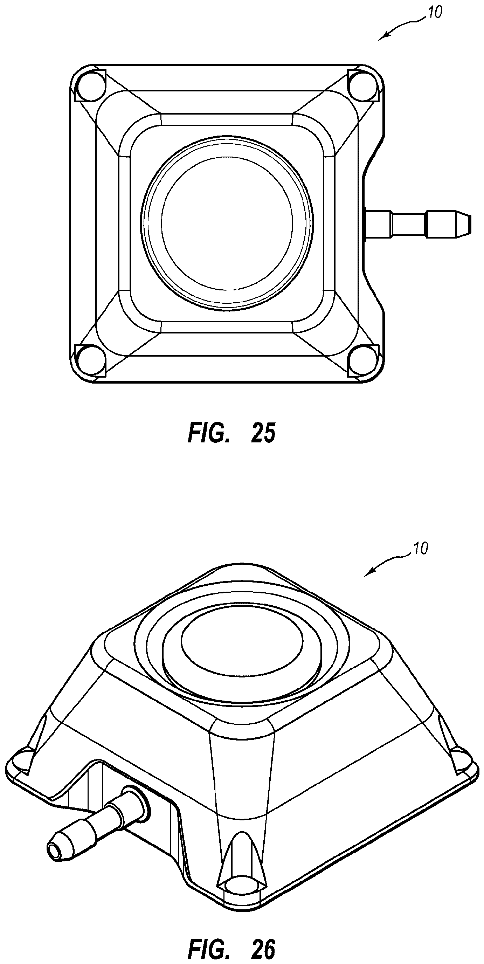

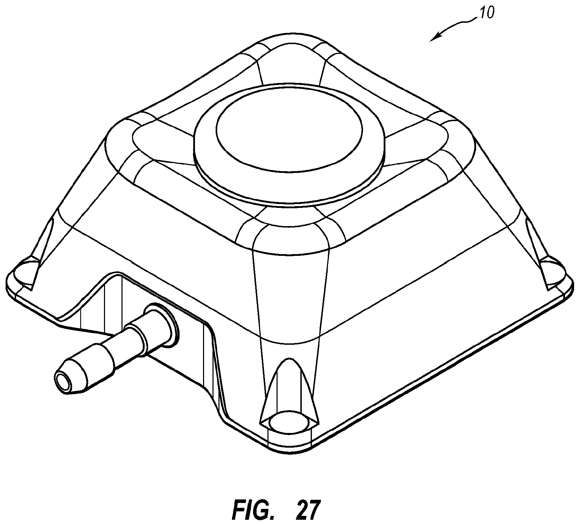

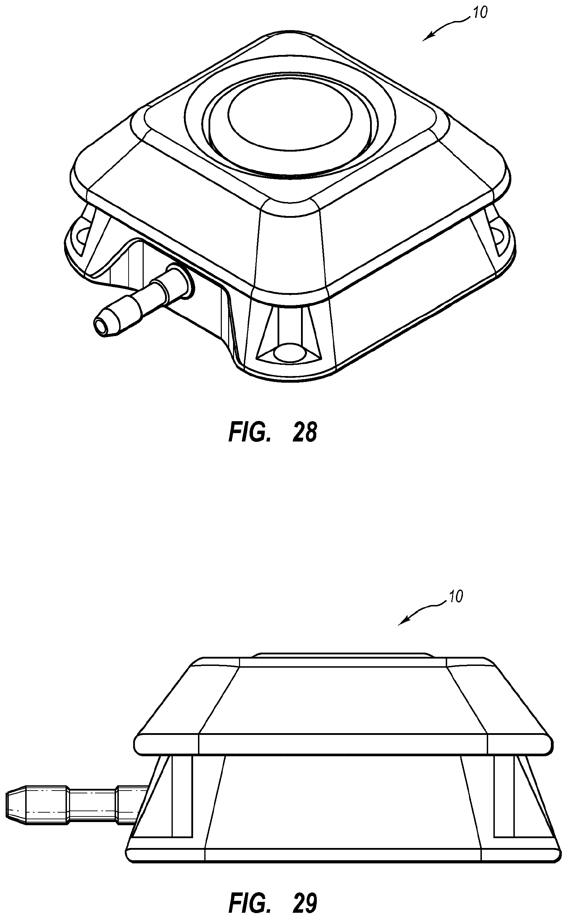

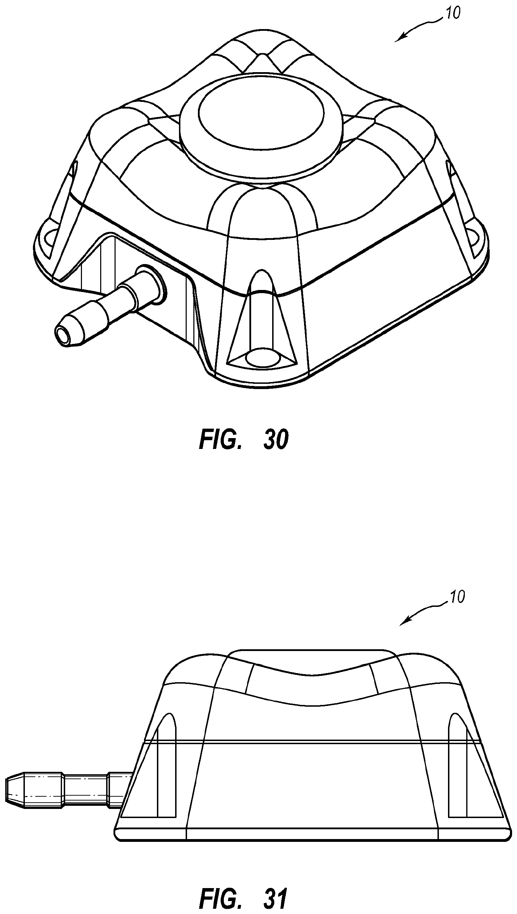

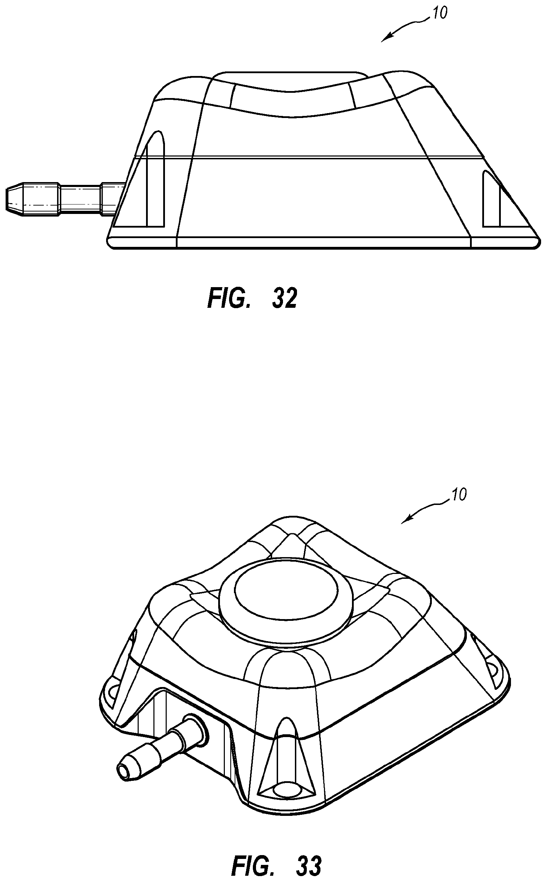

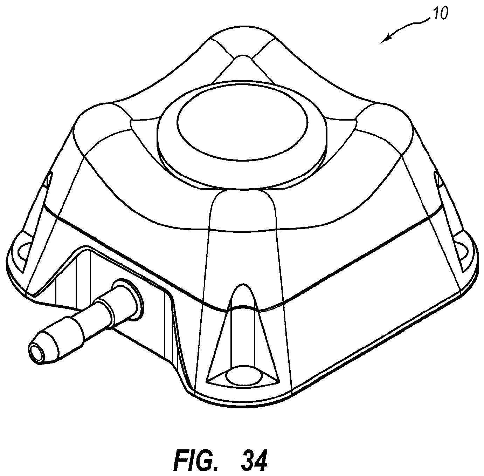

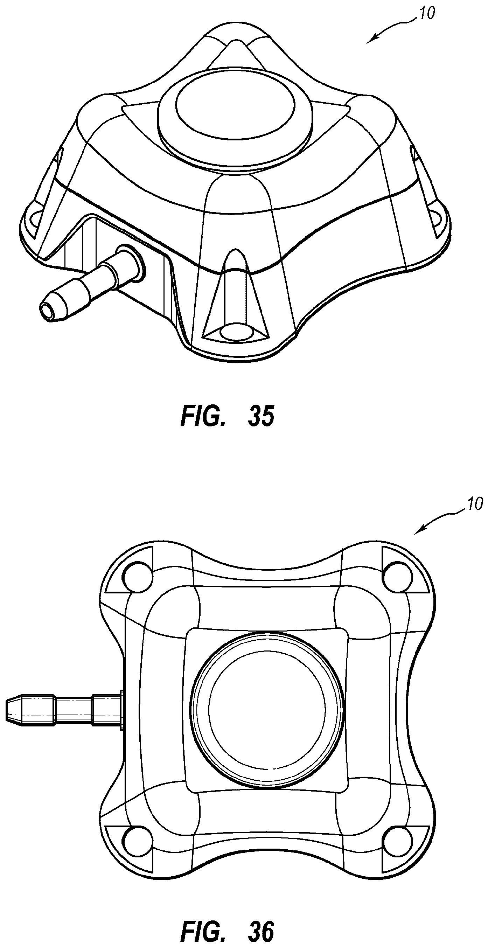

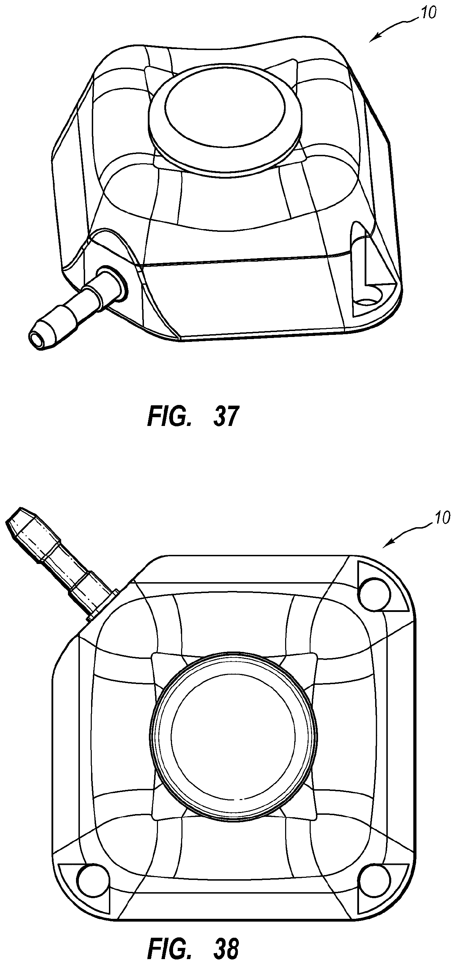

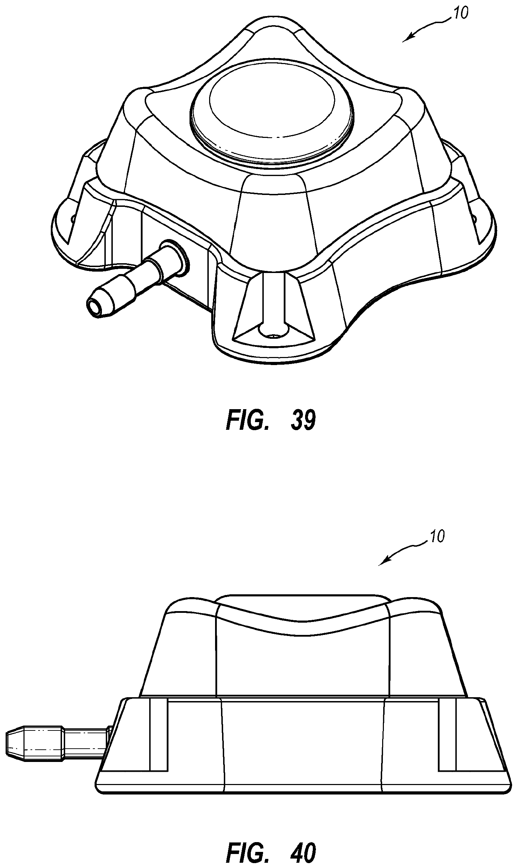

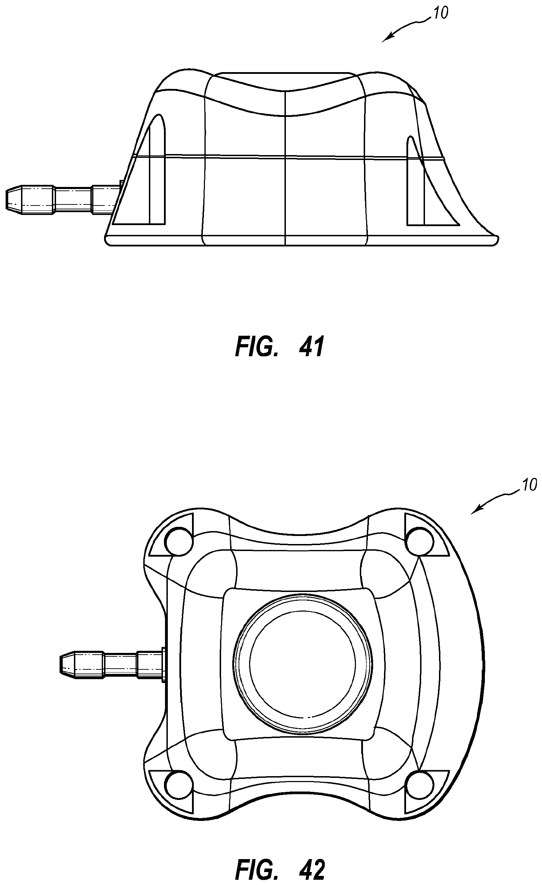

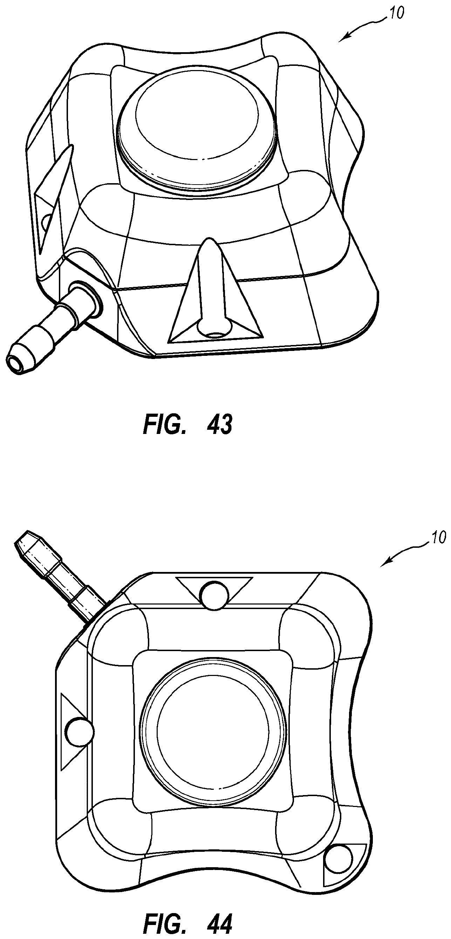

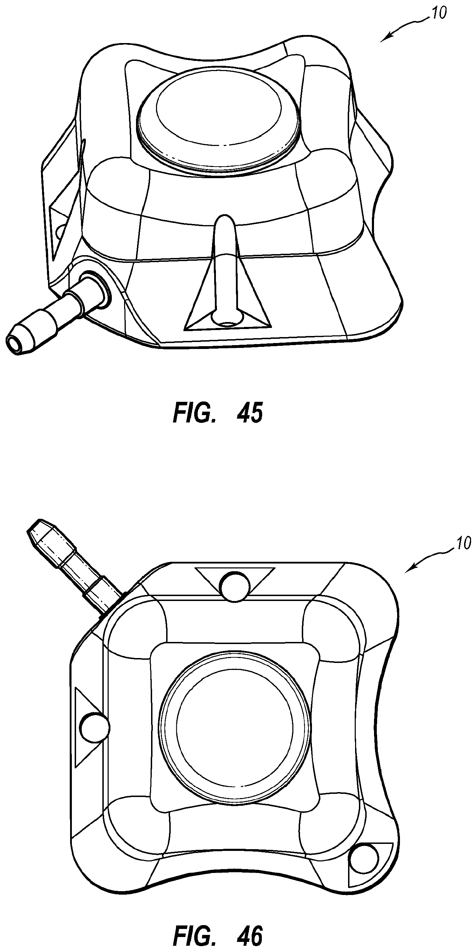

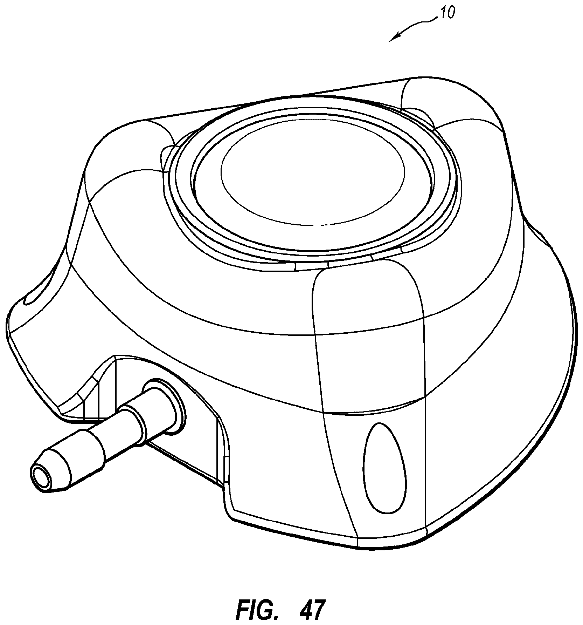

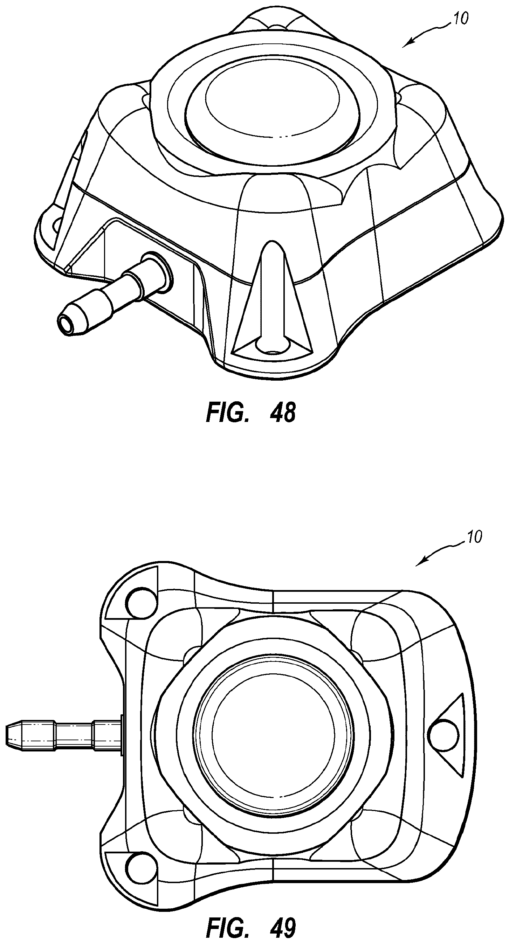

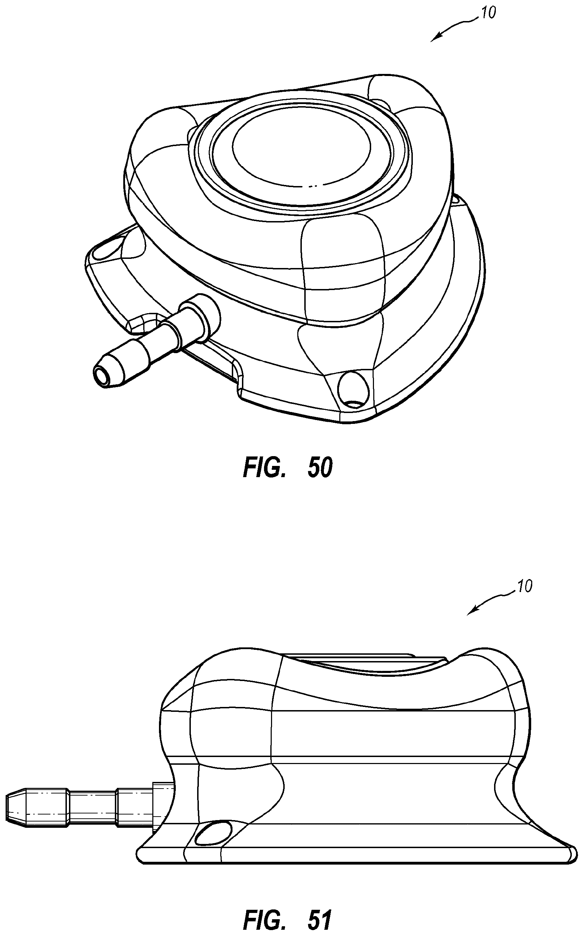

FIGS. 25-51 show perspective views of additional embodiments of an access port;
FIG. 52 shows a bottom perspective view of an access port according to one embodiment;
FIG. 53A shows a top view of the access port shown in FIG. 52;
FIG. 53B shows a bottom view of the access port shown in FIG. 52;
FIG. 54A represents a radiographic image of the access port shown in FIG. 52 when viewed from above the access port;
FIG. 54B represents a radiographic image of the access port shown in FIG. 52 when viewed at an angle of approximately 20 degrees;
FIG. 54C represents a radiographic image of the access port shown in FIG. 52 when viewed at an angle of approximately 50 degrees;
FIG. 55 shows a cross-sectional view of the access port shown in FIG. 52;
FIGS. 56A and 56B show cross-sectional views of example embodiments of engraved features on an access port surface;
FIG. 57A shows a top perspective view of an access port according to one embodiment;
FIG. 57B shows a bottom perspective view of the access port shown in FIG. 57A;
FIG. 57C shows a bottom view of the access port shown in FIG. 57A;
FIG. 58A shows a top perspective view of another embodiment of an access port;
FIG. 58B shows a bottom perspective view of the access port shown in FIG. 58A;
FIG. 58C shows a bottom view of the access port shown in FIG. 58A;
FIG. 59A shows a side view of an embodiment of an access port;
FIG. 59B shows a bottom view of the access port shown in FIG. 59A;
FIG. 60A shows a bottom perspective view of an additional embodiment of an access port;
FIG. 60B shows a bottom view of the access port shown in FIG. 60A;
FIG. 61A shows a bottom perspective view of an additional embodiment of an access port;
FIG. 61B shows a bottom view of the access port shown in FIG. 61A;
FIG. 62A shows a bottom view of an additional embodiment of an access port;
FIG. 62B shows a side view of the access port shown in FIG. 62A;
FIG. 62C shows an end view of the access port shown in FIG. 62A;
FIG. 63A shows a bottom view of another embodiment of an access port;
FIG. 63B shows a side view of the access port shown in FIG. 63A;
FIG. 63C shows an end view of the access port shown in FIG. 63A;
FIG. 64 shows a top view of an access port according to one embodiment;
FIG. 65 shows a perspective view of an access port according to one embodiment;
FIGS. 66A-66D show various views of an access port according to one embodiment;
FIG. 67 shows a bottom perspective view of an access port according to one embodiment;
FIGS. 68A-68C show various views of a septum of an access port according to one embodiment;
FIG. 69 shows a perspective view of an access port according to one embodiment;
FIG. 70 shows a perspective view of an identifier for an access port according to one embodiment;
FIG. 71 shows a top view of an identifier for an access port according to one embodiment;
FIG. 72 shows a perspective view of a port and a catheter including an identifier according to one embodiment;
FIGS. 73A and 73B show various views of an identifier for an access port according to one embodiment;
FIGS. 74A and 74B show views of an identifier for an access port according to one embodiment;
FIGS. 75A-75C show various views of an identifier for an access port according to one embodiment;
FIG. 76 is a view of an identifier for an access port according to one embodiment;
FIG. 77 is a perspective view of an access port including the identifier of FIG. 76;
FIG. 78 is a view of an identifier for an access port according to one embodiment;
FIGS. 79A-79C are simplified cross sectional views of placement of an identifier in a portion of an access port according to one embodiment;
FIGS. 80-81 are views of an identifier for an access port according to one embodiment;
FIG. 82 is a perspective view of an implantable access port configured in accordance with one embodiment;
FIGS. 83A and 83B are various exploded views of the access port of FIG. 82;
FIG. 84 is a top view of a radiopaque insert of the access port of FIG. 82;
FIGS. 85A and 85B are various views of a base portion and radiopaque insert of the access port of FIG. 82;
FIG. 86 is a bottom view of a cap portion of the access port of FIG. 82;
FIG. 87 is a bottom view of the cap portion of FIG. 86 with the radiopaque insert of FIG. 84 mated thereto; and
FIG. 88 is a bottom view of the access port of FIG. 82.

### Detailed Description

The instant disclosure relates generally to percutaneous access and, more specifically, to methods and devices associated with percutaneous access. Generally, the instant disclosure relates to an access port for subcutaneous implantation. In one embodiment, an access port may allow a physician or other medical personnel to obtain long term percutaneous access to the interior of a patient's body. Employing an access port for percutaneous access may reduce the opportunity for infection by inhibiting fluid connections (that extend into the interior of a patient's body) from the patient's skin and from the external environment. The access device allows access to the interior of the patient without requiring a needle to pierce the skin. Further, internal components, such as a catheter or a valve, may be replaced without a surgical procedure. Features or aspects of the instant disclosure may apply to any such access ports for subcutaneous access to a patient, without limitation. The access port may be injected by hand (e.g., via a syringe including a needle) for example, or may be injected and pressurized by mechanical assistance (e.g., a so-called power injectable port).

Power injectable ports may be employed in, among other processes, for example, computed tomography ("CT") scanning processes. More particularly, a so-called "power injector" system may be employed for injecting contrast media into a peripherally inserted intravenous (IV) line. For example, such power injectors or injection systems may be commercially available from Medrad, Inc., a subsidiary of Schering AG, Germany and may be marketed under the trademark STELLANT^{®}. Because fluid infusion procedures are often defined in terms of a desired flow rate of contrast media, such power injection systems are, in general, controllable by selecting a desired flow rate.

More specifically, the instant disclosure relates to an access port having at least one perceivable or identifiable feature for identifying the access port, wherein the identifiable feature is perceivable after the access port is implanted within a patient. For example, at least one or perhaps multiple identifiable feature(s) of an access port contemplated by the instant disclosure may be correlative to information (e.g., a manufacturer's model or design) pertaining to the access port. Thus, an identifiable feature from an access port of a particular model may be unique in relation to most if not all other identifiable features of another access port of a different models or design. Of course, the at least one identifiable feature of an access port contemplated by the instant disclosure may be further correlative with any information of interest, such as type of port, catheter type, date of manufacture, material lots, part numbers, etc. In one example, at least one identifiable feature of an access port may be correlative with the access port being power injectable. In this way, once at least one identifiable feature of an access port is observed or otherwise determined, correlation of such at least one feature of an access port may be accomplished, and information pertaining to the access port may be obtained.

In one embodiment, at least one feature may be perceived by palpation (i.e., to examine by touch), by way of other physical interaction, or by visual observation. Accordingly, a person of interest may touch or feel the access port through the skin to perceive at least one identifying characteristic thereof. In another embodiment, at least one identifiable feature may be perceived via x-ray or ultrasound imaging. In yet a further embodiment, at least one identifiable feature may be perceived through magnetic, light, or radio energy interaction or communication with the access port.

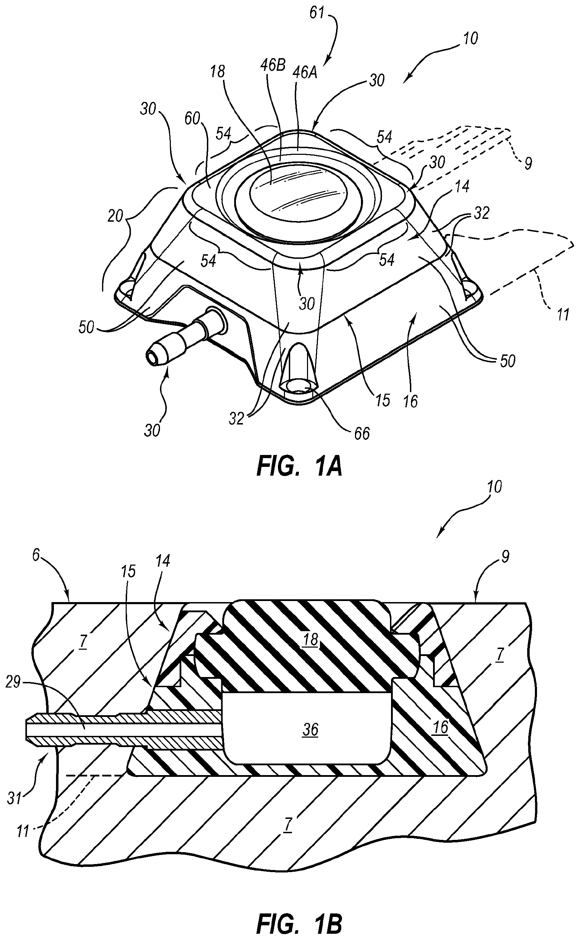

Turning to the embodiment wherein at least one feature may be perceived through palpation, other physical interaction, or visual observation, a topography or exterior surface feature of an access port contemplated by the instant disclosure may be configured for perception. For example, referring to FIGS. 1A and 1B, an exemplary access port 10 contemplated by the instant disclosure is shown. FIGS. 1A and 1B show a perspective view and a schematic side cross-sectional view, respectively, of an access port 10 for allowing percutaneous or otherwise internal access to a patient's body. Access port 10 includes a housing or body 20 defined by a cap 14 and a base 16. Cap 14 and base 16, as known in the art, may be configured for capturing therebetween a septum 18. As shown in FIG. 1A, cap 14 and base 16 may matingly engage one another along a mating line 15. Cap 14 and base 16 may be secured or affixed to one another via mechanical fasteners such as screws or other fastening devices, may be adhesively affixed to one another, or may be affixed to one another as known in the art. Further, cap 14, base 16, and septum 18 may collectively define a cavity 36 in fluid communication with a lumen 29 of outlet stem 31.

The body 20 may be implanted in a patient 7, as shown in FIG. 1B, to dispose the cavity 36 subcutaneously within the patient 7. Also, suture apertures 66 (FIG. 1A) may be used to affix the access port 10 within the patient 7, if desired. After the body 20 is implanted in a patient 7, the upper surface of the septum 18 may be substantially flush with the surface of the skin 6 of the patient 7 and may be repeatedly punctured for creating a percutaneous passageway from the exterior of the skin of the patient into the cavity 36. The outlet stem 31 may create a fluid-communicative passageway from the cavity 36 through the outlet stem 31 and into the interior of the patient 7. A catheter may be coupled to the outlet stem 31 for fluid communication with the cavity 36 and for transferring fluid from the cavity 36 to a desired remote location from the cavity 36 and within a patient 7.

Body 20 of access port 10 may comprise a bio-compatible material such as polysulfone, titanium, acetyl resin, or any other suitably bio-compatible material as known in the art. Accordingly, the body 20 may be formed from a bio-compatible plastic material. If desired, the body 20 may comprise a penetrable material for penetration by sutures or needles. In another embodiment, and as discussed further hereinbelow, body 20 may comprise an impenetrable material such as, for instance, a metal if desired. Body 20 may include a concave bottom or, in another embodiment, may include a flat bottom, without limitation.

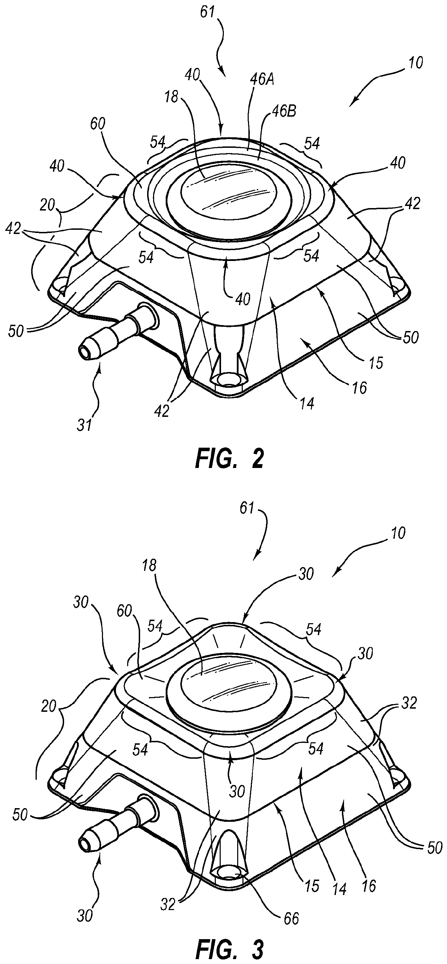

According to the instant disclosure, access port 10 may comprise a body 20 exhibiting at least one identifiable feature. More particularly, as shown in FIG. 1A, body 20 may exhibit a partial generally pyramidal shape (i.e., a polygonal base having surfaces for each side of the polygon extending toward a common vertex otherwise known as a frustum). Generally, a body 20 of an access port 10 may exhibit a partial pyramidal shape extending between a generally quadrilateral shaped base positioned at reference plane 11 and a generally quadrilateral shaped upper base positioned at reference plane 9. Reference planes 9 and 11 will not be shown in FIGS. 2-21, for clarity; however, reference to planes 9 or 11 with respect to FIGS. 2-21, as used herein, will refer to corresponding reference planes analogous to reference planes 9 and 11 as shown in FIGS. 1A and 1B.

As shown in FIG. 1A, the exterior of access port 10 is substantially defined by four substantially planar side surfaces 50 connected to one another by radiuses 32. In addition, the upper topography 61 of access port 10 is defined by upper surface 60 in combination with chamfers 46A and 46B and may be further defined by the upper surface of septum 18. Explaining further, the outer periphery of upper topography 61 may be described as a generally quadrilateral exterior formed by side regions 54 and having rounded corner regions 30 adjacent side regions 54. Such a configuration may provide an access port having at least one feature that may be perceived by palpation.

It may be appreciated that there are many variations to the geometry of access port 10 as shown in FIG. 1A. For instance, while the body 20 of access port 10 may be described as a partially pyramidal shape or frustum, the instant disclosure is not so limited. Rather, one or more of side surfaces 50 may be oriented at as may be desired, without reference to any other side surfaces 50. Accordingly, for example, one of surfaces 50 may be substantially vertical while the remaining surfaces 50 may be oriented at respective, selected angles. Furthermore, it should be understood that FIG. 1A is merely exemplary and that the dimensions and shape as shown in FIG. 1A may vary substantially while still being encompassed by the instant disclosure.

FIG. 2 shows a perspective view of another embodiment of access port 10 according to the instant disclosure. As shown in FIG. 2, the exterior of access port 10 is substantially defined by a generally parallelogram-shaped base (positioned at reference plane 11 as shown in FIGS. 1A and 1B) extending generally pyramidally to a generally parallelogram-shaped upper surface (positioned at reference plane 9 as shown in FIGS. 1A and 1B). As shown in FIG. 2, radiuses 42 may be larger than radiuses 32 as shown in FIG. 1A. Furthermore, the upper topography 61 of access port 10 as shown in FIG. 2 may include rounded corner regions 40 which are larger than rounded corner regions 30 as shown in FIG. 1A. Thus, FIG. 2 shows an exemplary embodiment of an access port 10 that may be perceivably distinguishable from access port 10 as shown in FIGS. 1A and 1B. For example, a difference between one exterior of an access port contemplated by the instant disclosure and another exterior of a different access port contemplated by the instant disclosure may be determined by way of palpation.

In another embodiment, in another aspect contemplated by the instant disclosure, a template may be employed for perceiving at least one feature of an access port. For instance, a complementarily-shaped template may be positioned over and abutted against an access port contemplated by the instant disclosure so as to determine if the access port matches or substantially corresponds to the shape of the template. Such a process may reliably indicate or perceive at least one feature of an access port contemplated by the instant disclosure. Of course, a plurality of templates corresponding to different models of access ports may be serially engaged with an unknown access port so as to perceive at least one feature thereof. Such a process may allow for identification (e.g., of a model or manufacturer) of an access port contemplated by the instant disclosure.

In another aspect contemplated by the instant disclosure, an upper topography of an access port may include at least one feature for identifying the access port. For example, as shown in FIG. 3, upper surface 60 of access port 10 may be nonplanar. More specifically, upper surface 60 may be tapered or may arcuately extend downwardly (i.e., toward reference plane 11 as shown in FIGS. 1A and 1B) as it extends radially inwardly toward septum 18. Otherwise, access port 10, as shown in FIG. 3, may be configured substantially as described hereinabove with reference to FIGS. 1A and 1B. Thus, upper surface 60 is one exemplary example of at least one perceivable feature for identification of an access port contemplated by the instant disclosure.

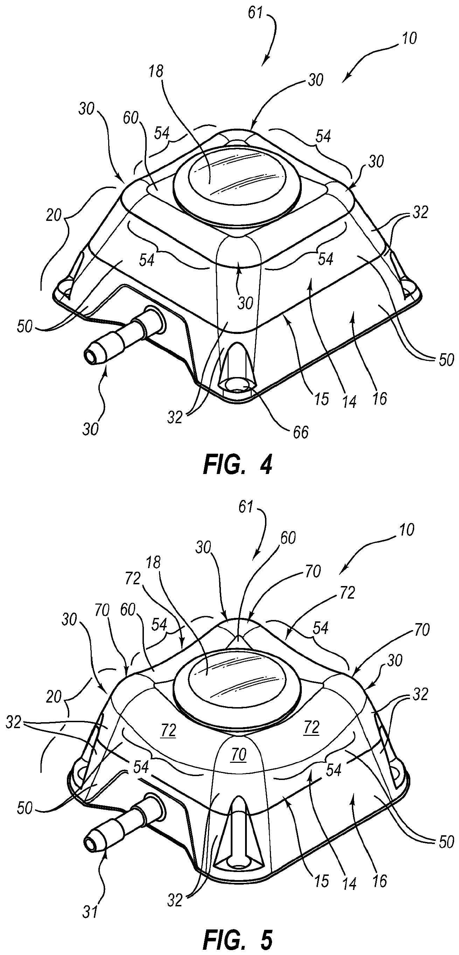

In yet a further embodiment of an access port contemplated by the instant disclosure, side regions 54 extending between rounded corner regions 30 may exhibit at least one perceivable feature. For example, as shown in FIG. 4, access port 10 may include one or more side regions 54 that extend arcuately between adjacent rounded corner regions 30. Otherwise, access port 10, as shown in FIG. 4, may be configured substantially as described hereinabove with reference to FIGS. 1A and 1B. Side regions 54 may be congruent or symmetric with respect to one another or, in another embodiment, may be configured differently with respect to one another, without limitation.

FIG. 5 shows a further exemplary embodiment of an access port contemplated by the instant disclosure. More specifically, access port 10, as shown in FIG. 5, includes side regions 54 that form recessed regions 72 between adjacent rounded corner regions 30. Put another way, the upper topography 61 may include alternating recessed regions 72 and protruding regions 70 positioned generally about a periphery of septum 18. Otherwise, access port 10, as shown in FIG. 5, may be configured substantially as described hereinabove with reference to FIGS. 1A and 1B. Such a configuration may provide an access port having at least one identifiable feature.

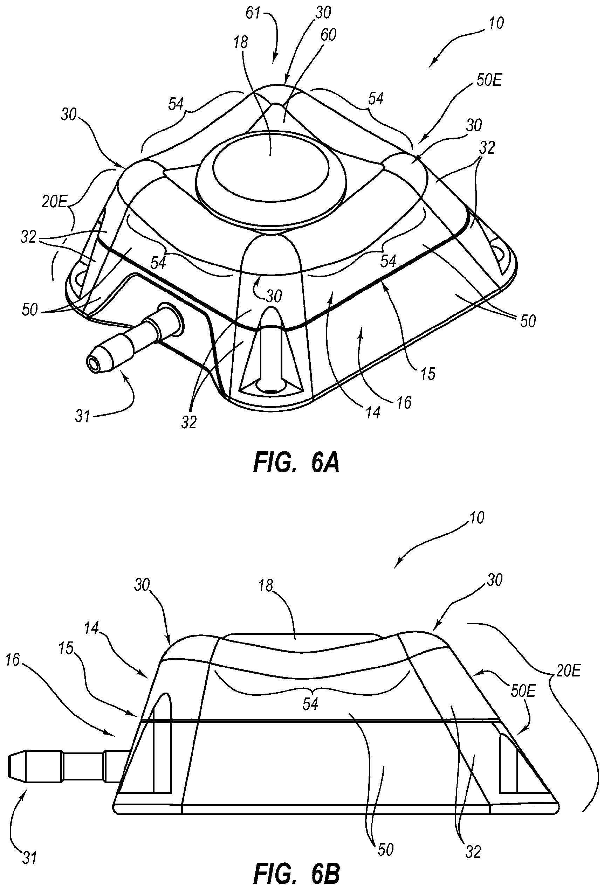

In a further embodiment of an access port contemplated by the instant disclosure, FIGS. 6A and 6B show a perspective view and a side view, respectively, of an access port 10 generally configured as is described with reference to FIG. 5 but having an elongated body 20E. More specifically, elongated body 20E of access port 10, as shown in FIGS. 6A and 6B, includes a side surface 50E that extends generally from upper topography 61 downwardly (i.e., toward reference plane 11 as shown in FIGS. 1A and 1B) and having a slope (e.g., an angle with respect to a vertical axis normal to an upper surface of septum 18) which is different from the other side surfaces 50. Otherwise, access port 10, as shown in FIG. 6, may be configured substantially as described hereinabove with reference to FIGS. 1A and 1B. Such a configuration may provide an elongated body 20E of an access port 10 having an elongated side portion.

Of course, one or more side surfaces of an access port according to the instant disclosure may be configured for forming a body exhibiting a selected shape as may be desired. An elongated body portion of an access port contemplated by the instant disclosure may form, in combination with other features as described hereinabove or, in another embodiment, taken alone, at least one perceivable feature for identification of an access port according to the instant disclosure.

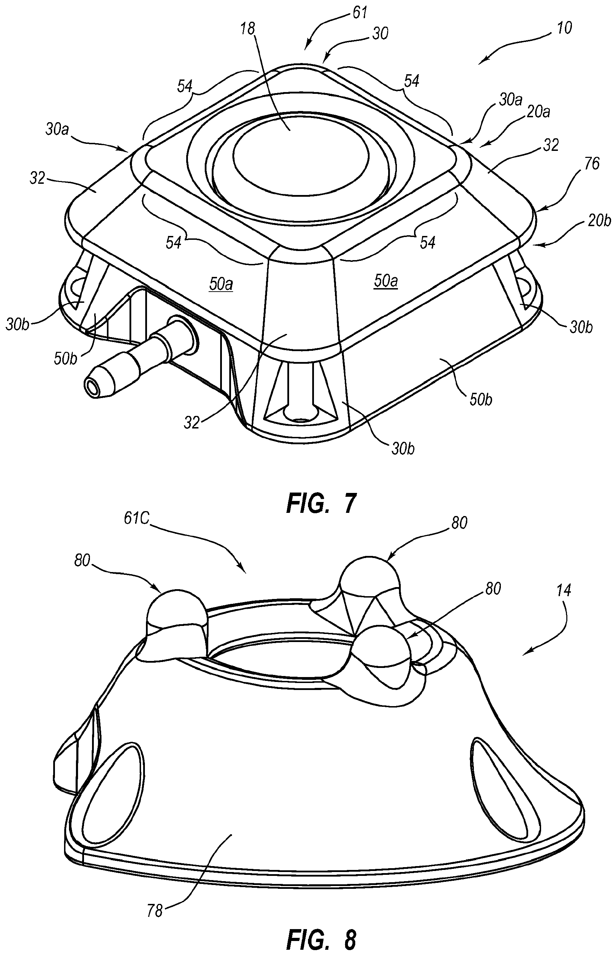

FIG. 7 shows a further embodiment of an access port encompassed by the instant disclosure. Particularly, as shown in FIG. 7, access port 10 may include an upper body portion 20a and a lower body portion 20b. Furthermore, each of upper body portion 20a and lower body portion 20b may exhibit a partial pyramidal shape (i.e., a frustum), wherein the body portions 20a and 20b are stacked vertically with respect to one another. Accordingly, upper body portion 20a may form an overhanging rim feature 76 extending along a periphery of access port 10. Explaining further, lower body portion 20b may have an exterior substantially defined by side surfaces 50b and rounded corner regions 30b, while upper body portion 20a may have an exterior substantially defined by side surfaces 50a, rounded corner regions 30a, and upper topography 61. It may be appreciated that overhanging rim feature 76 may be sized and configured for perception via palpation. Such a configuration may provide a suitable access port for delivery of a beneficial or medicinal substance, the access port being identifiable (e.g., by model number, manufacturer, etc.) after implantation.

It should be understood that the instant disclosure contemplates access ports having an exterior geometry that is not quadrilateral in nature. Rather, the instant disclosure contemplates that an access port may have an exterior which is generally cylindrical, generally conical, generally elliptical, generally oval, or an exterior that is otherwise arcuate in nature. Specifically, the instant disclosure contemplates that an access port having a substantially rounded or arcuate exterior may include at least one feature configured for identification of the access port after implantation. For example, as shown in FIG. 8, shows a cap 14 that exhibits an exterior surface 78 that is substantially conical. Cap 14 may be assembled to a suitable base (not shown) for capturing a septum (not shown) as described hereinabove to form an access port 10 as generally described with reference to FIGS. 1-7.

The instant disclosure further contemplates that at least one protrusion, protruding region, recess, recessed region, undulation, or adjacent features of different elevation may comprise a feature for identifying an access port contemplated by the instant disclosure. More specifically, upper topography 61C, as shown in FIG. 8, may include a plurality of protrusions 80. Protrusions 80 may exhibit partially spherical upper surfaces that transition into a lower portion of cap 14. In further detail, protrusions 80 may be circumferentially spaced about the periphery of septum (not shown) as may be desired. In one embodiment, a plurality of protrusions 80 may be symmetrically circumferentially spaced about the periphery of septum (not shown). More generally, at least one protrusion 80 may be sized, configured, and positioned for forming at least one identifiable feature of an access port. Of course, at least one protrusion 80 may be structured for facilitating comfort of a patient within which the access port is implanted. As may be appreciated, at least one protrusion 80 or more than one protrusion 80 may be included in an upper topography 61C of an access port (not shown) contemplated by the instant disclosure.

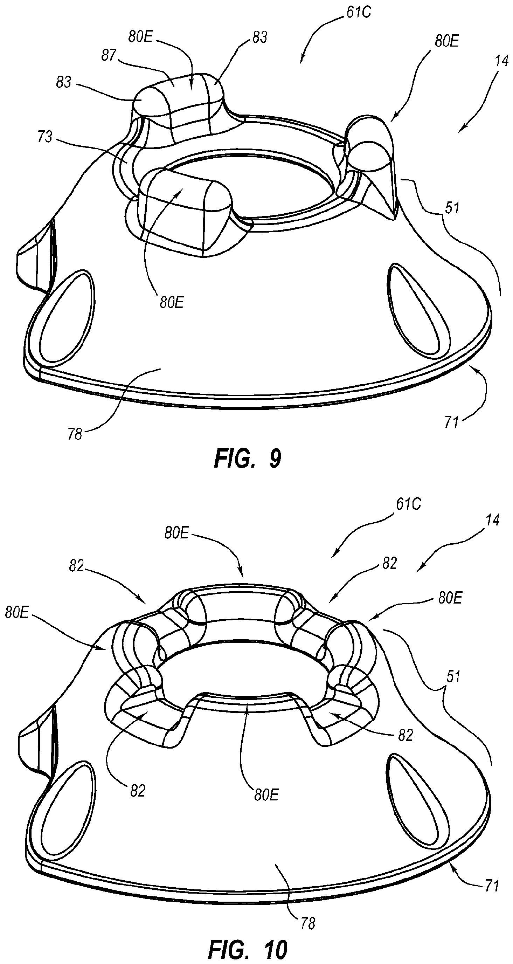

FIG. 9 shows another embodiment of a cap 14 including at least one protrusion 80E for forming and identifying an access port contemplated by the instant disclosure after implantation thereof within a patient. Protrusions 80E may extend circumferentially about a center of revolution. Thus, protrusions 80E may exhibit a body 87 portion circumferentially extending between rounded ends 83. Further, cap 14 may have an exterior surface 78 that is substantially symmetric about an axis of revolution. More generally, body 20 may extend from a generally circular, generally elliptical, or generally oval base positioned at a lower extent 71 of the cap 14 to an upper generally circular, generally elliptical, or generally oval cross section that is smaller than a cross section of the base and is positioned at an upper extent 73 (without considering protrusions 80E) of the cap 14. In addition, side surface 51, as shown in FIG. 9, extends arcuately between the base and the upper topography 61 of cap 14. Side surface 51 may extend in a generally tapered or conical fashion, may exhibit a radius or other arcuate shape, or may otherwise transition between a cross section of the base of the access port to a cross section proximate the upper topography 61C thereof.

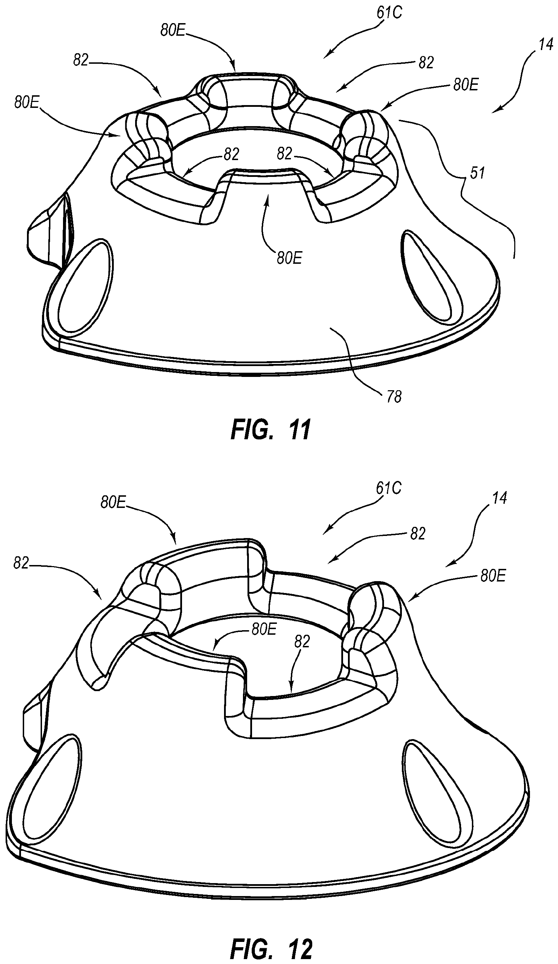

Further, FIG. 10 shows an embodiment of a cap 14 for forming an access port contemplated by the instant disclosure having an upper topography 61C thereof comprising alternating circumferentially extending protrusions 80E and circumferentially extending recesses 82, wherein the circumferentially extending protrusions 80E are circumferentially larger than the circumferentially extending recesses 80E. In another embodiment of an access port contemplated by the instant disclosure, FIG. 11 shows a perspective view of a cap 14 having an upper topography 61C thereof comprising alternating circumferentially extending protrusions 80E and circumferentially extending recesses 82, wherein the circumferentially extending protrusions 80E and the circumferentially extending recesses 82 are substantially equal in (circumferential) sized or extension. In yet a further embodiment of a cap 14 for forming an access port contemplated by the instant disclosure, FIG. 12 shows a perspective view of a cap 14 having an upper topography 61C thereof comprising three circumferentially extending protrusions 80E and three circumferentially extending recesses 82, arranged so as to alternate circumferentially, wherein the circumferentially extending protrusions 80E and the circumferentially extending recesses 82 are substantially equal in (circumferential) size.

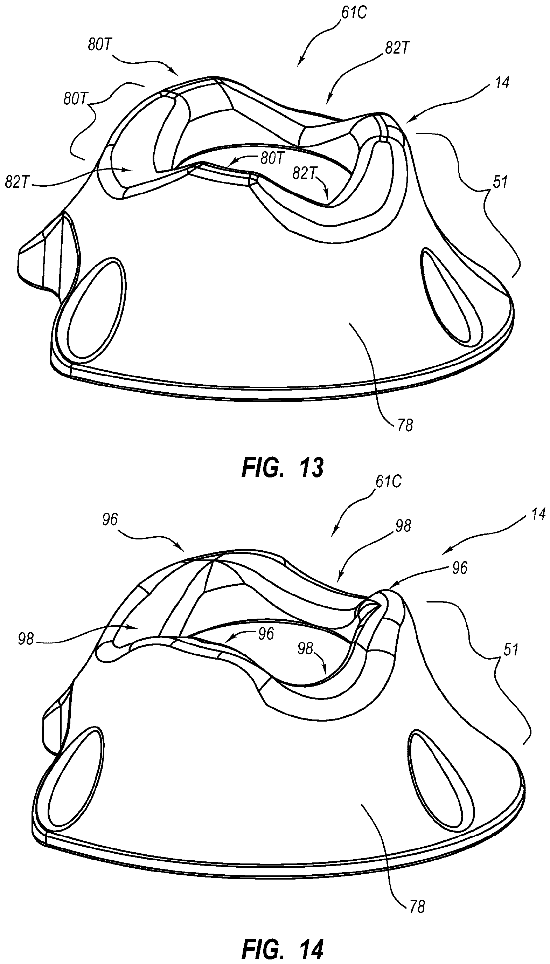

FIG. 13 shows a perspective view of an additional embodiment of an cap 14 for forming an access port contemplated by the instant disclosure including an upper topography 61C including circumferentially extending protrusions 80T and circumferentially extending recesses 82T, wherein transition regions 81 are provided between circumferentially extending protrusions 80T and circumferentially extending recesses 82T. Such transition regions 81, as shown in FIG. 13, may taper or generally smoothly transition between a circumferentially extending protrusion 80T and a circumferentially extending recess 82T. Also, FIG. 14 shows a perspective view of an additional embodiment of a cap 14 for forming an access port contemplated by the instant disclosure including an upper topography 61C including protrusion regions 96 and recessed regions 98 that transition between one another and alternate circumferentially so as to form an undulating topography comprising upper topography 61C. Such an undulating topography, as shown in FIG. 14, generally smoothly transitions between circumferentially adjacent protrusion regions 96 and recessed regions 98.

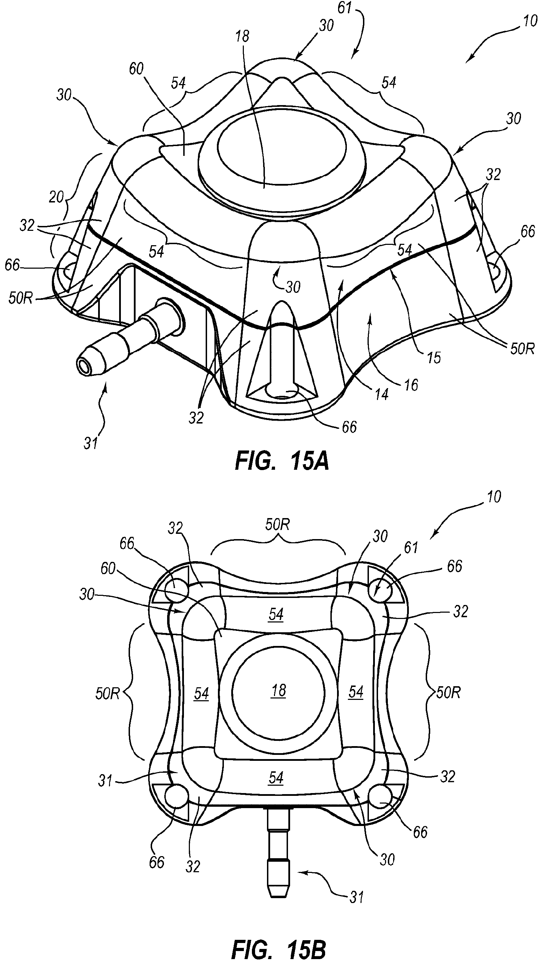

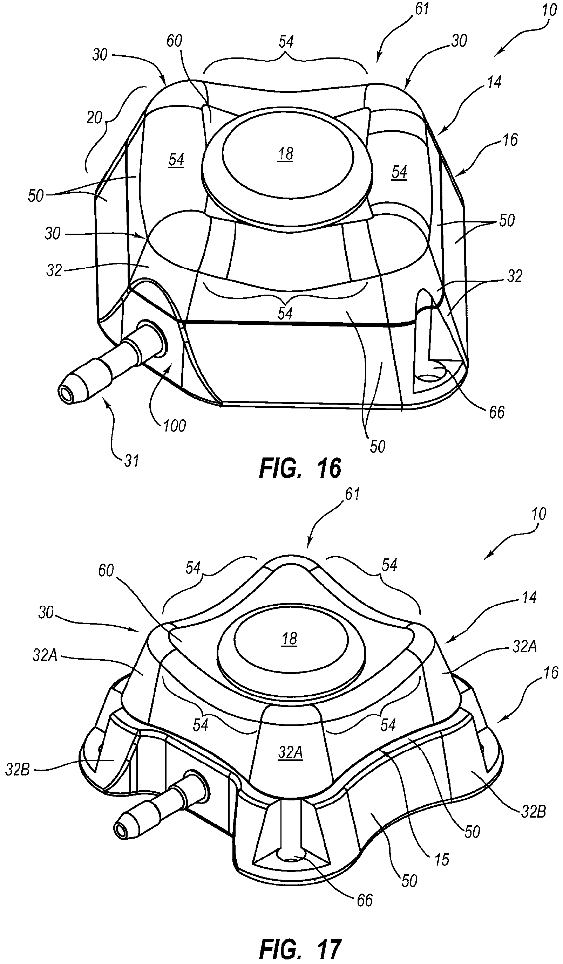

In a further embodiment of an access port contemplated by the instant disclosure, FIG. 15A and 15B show a perspective view and a top elevation view, respectively, of an access port 10 generally configured as is described with reference to FIG. 5 but may include at least one nonplanar side surface. In another embodiment, access port 10 as shown in FIG. 15 may be configured as shown in FIGS. 1-4 or FIGS. 6-7, or any embodiments described hereinbelow, without limitation. More specifically, elongated body 20 of access port 10, as shown in FIGS. 15A and 15B, includes three side surfaces 50R that extend arcuately (as shown in FIG. 15B). Such a configuration may provide an access port 10 that is identifiable subsequent to implantation. In yet another embodiment of an access port contemplated by the instant disclosure, FIG. 16 shows a perspective view of an access port 10 including a side wall 100 that truncates a portion of a radius 32 formed between side surfaces 50 of access port 10. It may also be noted that such an access port 10 may include three suture apertures 66, which may, taken alone or in combination with at least one other feature, comprise at least one identifiable feature of an access port contemplated by the instant disclosure. In addition, as shown in FIG. 16, outlet stem 31 may extend from side wall 100.

In a further embodiment of an access port contemplated by the instant disclosure, FIG. 17 shows a perspective view of an access port 10 wherein cap 14 and base 16, when assembled to one another along mating line 15, form a flange feature or lip feature 102 that extends about at least a portion of the periphery of the access port 10. As shown in FIG. 17, lip feature 102 extends substantially about the periphery of the access port 10, proximate to the mating line 15 between cap 14 and base 16. Such a feature may comprise at least one identifiable feature of an access port contemplated by the instant disclosure. Thus, it may be appreciated that a peripheral discontinuity between the cap 14 and base 16 may be formed generally along the mating line 15 therebetween. In the embodiment of an access port as shown in FIG. 7, an overhanging rim feature 76 may comprise a peripheral discontinuity or, in the embodiment of an access port as shown in FIG. 17, a lip feature 102 may comprise a peripheral discontinuity.

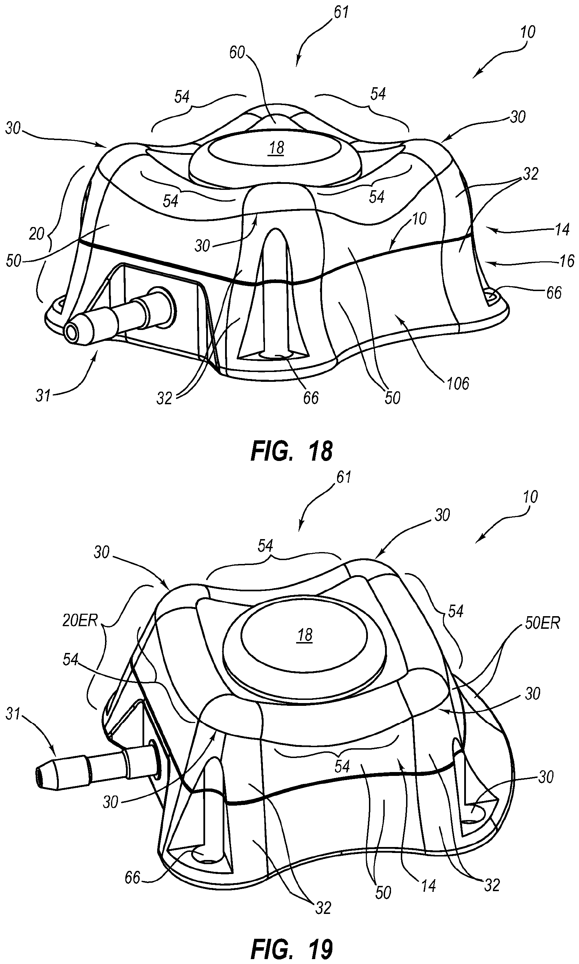

In a further embodiment of an access port contemplated by the instant disclosure, FIG. 18 shows a perspective view of an access port 10 wherein at least a portion of at least one side surface 50 is concave. As shown in FIG. 18, concave region 106 of side surface 50 is concave. Concavity (i.e., a concave region 106) may be exhibited over at least a portion of a side surface of an access port of any of the embodiments as shown herein, without limitation. Thus, at least one side surface 50 of an access port contemplated by the instant disclosure having at least at least a portion thereof that is concave is one exemplary example of at least one perceivable feature for identification of an access port contemplated by the instant disclosure.

In a further embodiment of an access port contemplated by the instant disclosure, FIG. 18 shows a perspective view of an access port 10 wherein at least a portion of at least one side surface 50 is concave. As shown in FIG. 18, region 106 of side surface 50 is concave. Concavity may be exhibited over at least a portion of a side surface of an access port of any of the embodiments as shown herein, without limitation. Thus, at least one side surface 50 of an access port contemplated by the instant disclosure having at least at least a portion thereof that is concave is one exemplary example of at least one perceivable feature for identification of an access port contemplated by the instant disclosure.

In a further embodiment of an access port contemplated by the instant disclosure, FIG. 19 shows a perspective view of an access port 10 generally configured as is described with reference to FIG. 6A and 6B. More specifically, elongated body 20ER, as shown in FIG. 19 includes a side surface 50ER that extends arcuately from upper topography 61 of access port 10 downwardly (i.e., toward reference plane 11 as shown in FIGS. 1A and 1B). Such a configuration may provide an elongated body 20E of an access port 10 having an elongated side portion.

It should be understood from the above-described various embodiments of an access port contemplated by the instant disclosure that many variations, additions, or different features may be encompassed by the instant disclosure. Thus, the instant disclosure is not limited to the several above-described exemplary embodiments.

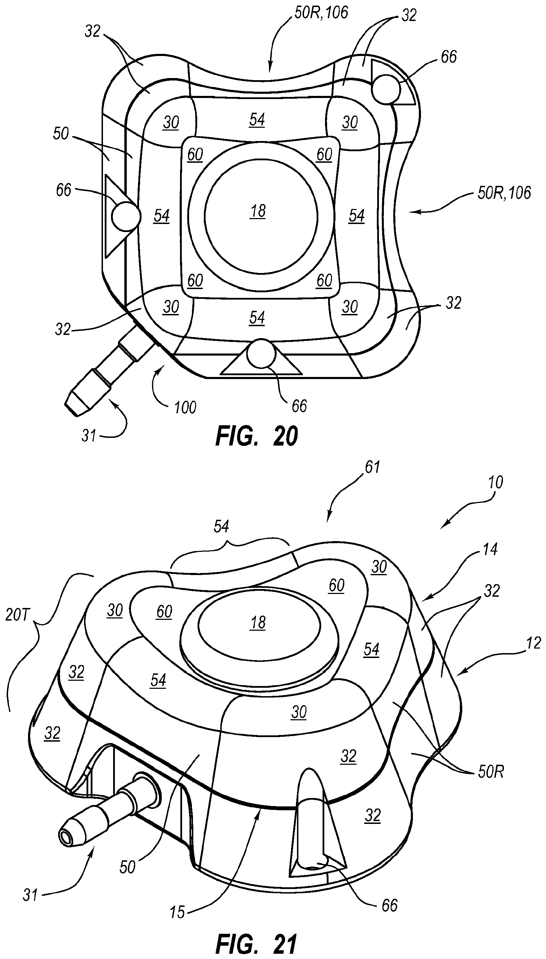

For example, as shown in FIG. 20, which shows a top elevation view of an access port 10 contemplated by the instant disclosure, an access port 10 may include a side wall 100 that at least partially truncates a radius 32 between side surfaces 50, outlet stem 31 extending from side wall 100, and at least one of a concave region 106 and an arcuate surface 50R. Further, as shown in FIG. 20, suture apertures 66 may be positioned so as to identify the access port 10 after subcutaneous implantation.

Additionally, the instant disclosure contemplates access ports having an exterior geometry that is polygonal in nature. Specifically, the instant disclosure contemplates that an access port contemplated by the instant disclosure may exhibit a generally triangular exterior. Thus, as shown in FIG. 21, body 20 may exhibit a generally pyramidal or tapered shape (i.e., a polygonal base having surfaces for each side of the polygon extending toward a common vertex). Generally, a body 20T of an access port 10 may extend between a generally triangularly-shaped base and a relatively smaller, generally triangularly-shaped upper base. Accordingly, the exterior of access port 10 may be substantially defined by three side surfaces (e.g., 50, 50R, 102, 50E) having radiuses 32 extending therebetween. In addition, the upper topography 61 of access port 10 may be defined by upper surface 60 in combination with side regions 54 and rounded corner regions 30. Such a configuration may provide an access port having at least one feature that may be perceived by palpation.

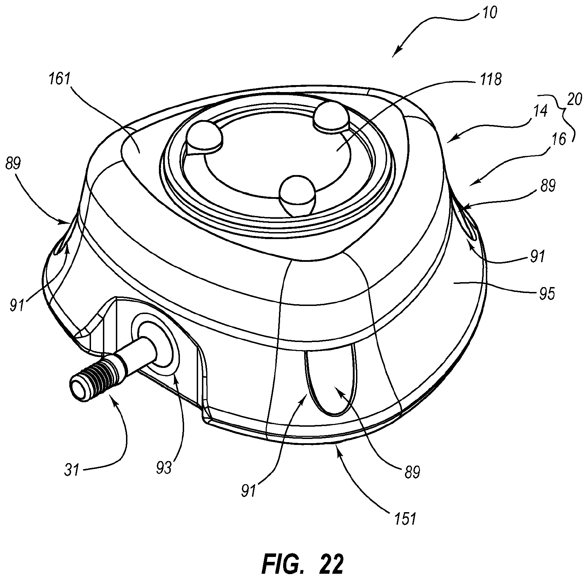

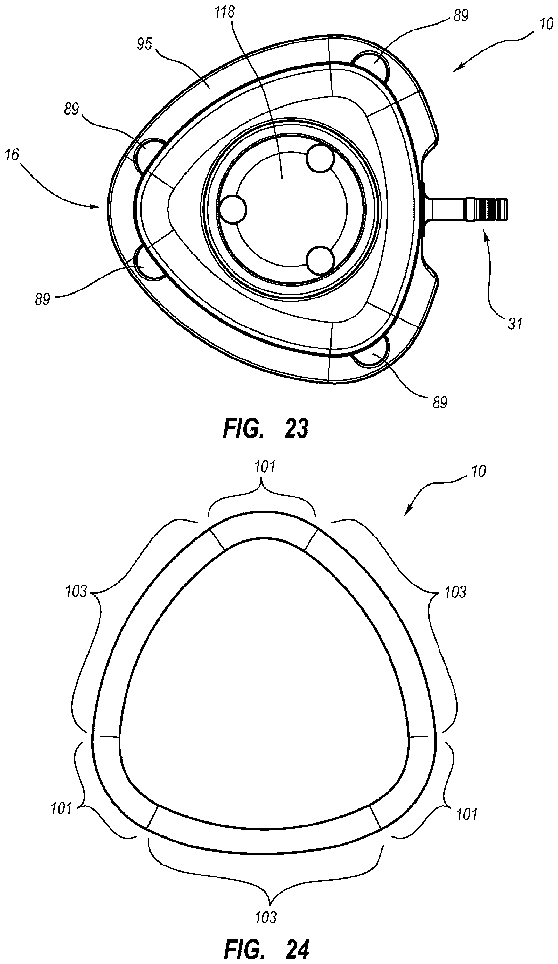

FIGS. 22 and 23 show a perspective view and a top elevation view of another embodiment of an access port including a generally triangular exterior geometry. More particularly, as shown in FIGS. 22 and 23, a cap 14 and base 16 (collectively forming a housing) may capture a septum 118 to form an access port 10. Further, outlet stem 31 may include a stem base that may be positioned within and sealed to an outlet recess 93 formed within base 16. The outlet stem 31 may be in fluid communication with a cavity formed within the access port 10. Optionally, suture plugs 89 may be positioned within suture cavities 91 formed in base 16. Suture plugs 89 may comprise a pliant material (e.g., silicone, rubber, etc.) that may provide some resilience between sutures coupling the access port 10 (i.e., the base 16) to a patient. In further detail, a side periphery 95 (e.g., one or more side walls) of access port 10 may be generally triangular. Thus, cap 14 and base 16 may collectively form a generally triangular housing or body of access port 10. Also, the instant disclosure contemplates that side periphery 95 may increase or decrease in cross-sectional size (e.g., by tapering or arcuately transforming) between upper surface 161 of cap 14 and lower surface 151 of base 16. As shown in FIGS. 22 and 23, a transverse cross section (taken in a selected plane substantially parallel to lower surface 151 of base 16) of access port 10 may be larger proximate to lower surface 151 of base 16 and may be relatively smaller proximate upper surface 161 of cap 14.

Additionally, FIG. 24 shows a simplified representation of a transverse cross section of access port 10. As shown in FIG. 24, side periphery 95 of access port 10 may define three side regions 103 that extend between associated vertex regions 101. In addition, in one embodiment and as shown in FIG. 24, side periphery 95 may define a substantially equilateral generally triangular shape. As one of ordinary skill in the art will appreciate, side regions 103 may arcuately extend between associated vertex regions 101; thus, side regions 103 may form "sides" of a generally triangular shape. Further, although vertex regions 101 are rounded, it may be appreciated that such vertex regions 101 form an intersection between adjacent side regions 103. Accordingly, one of ordinary skill in the art will appreciate that the phrase "generally triangular," as used herein, encompasses any generally three-sided geometry wherein adjacent sides intersect, without limitation. For example, the phrase "generally triangular" encompasses three sided polygons, circular triangles, equilateral triangles, etc., without limitation.

The instant disclosure also contemplates that at least one feature of an access port contemplated by the instant disclosure may not be observable visually or by palpation but, rather, may be otherwise observable. For example, the instant disclosure contemplates that at least one feature of an access port may be observable through interaction with an imaging technology such as x-ray or ultrasound. For example, in one embodiment, a metal feature (e.g., a plate or other metal geometry) may be included by an access port contemplated by the instant disclosure. As may be appreciated, such a metal feature may be represented on an x-ray generated by exposure of the access port to x-ray energy while simultaneously exposing x-ray sensitive film to x-ray energy passing through the access port. Further, the instant disclosure contemplates that a size, shape, or both size and shape of a metal feature of an access port may be configured for enhancing identification of an access port. For example, assuming that a metal feature comprises a metal plate, a size, shape, or both may be selectively tailored for identification of an access port. Similarly, a feature of an access port contemplated by the instant disclosure may be tailored for detection via ultrasound interaction. Such a feature may comprise an exterior topographical feature. In another embodiment, such a feature may comprise a composite structure including two or more materials that form an interface surface that may be identified by ultrasound imaging.

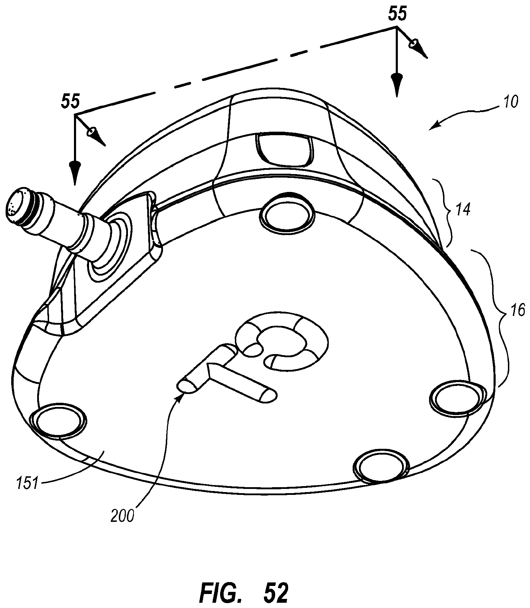

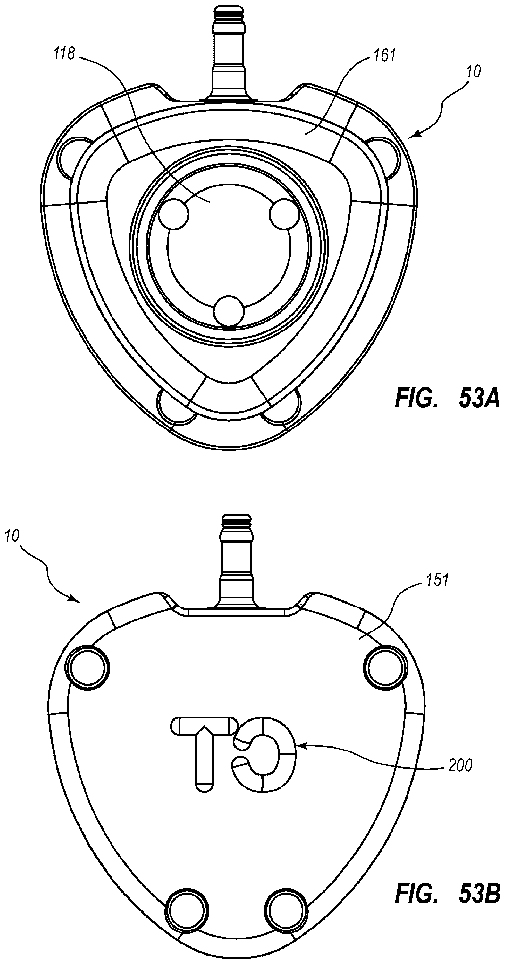

One example embodiment of a feature observable through interaction with imaging technology contemplated by the instant disclosure is shown in FIGS. 52, 53A, and 53B. FIG. 52 depicts a bottom perspective view of an access port 10. FIG. 53A shows a top view of the access port 10, while FIG. 53B shows a bottom view of the access port. The access port 10 of FIGS. 52, 53A, and 53B is similar in some respects to the access port 10 as seen in FIGS. 22 and 23, including a cap 14 and a base 16 that cooperate to define a body. In the present example embodiment, however, the lower surface 151 of the base 16 includes an identification feature 200, as seen in FIGS. 52 and 53B. It is contemplated that the identification feature 200 can be one or more alphanumeric characters, such as the "CT" depicted. Additionally, the instant disclosure contemplates the use of other markings, such as one or more symbols, patterns, characters, designs, a combination thereof, etc. The identification feature 200 can be of any size, shape, or both in order to tailor the identification feature for the specific identification of one or more of a variety of characteristics of the access port. Specifically, in one embodiment the identification feature 200 can convey information to a practitioner regarding the power-injectability of the implanted access port. Note that in the present embodiment, the identification feature 200 is defined as a recessed feature, whereas in other embodiments the identification feature may be defined in other ways, as discussed hereafter.

As mentioned above, FIG. 53A depicts a top view of the access port 10. Note that the identification feature 200 is not observable through the upper surface 161 of the cap 14 or through the septum 118 without the interaction of imaging technology. As seen in FIG. 53B, the alphanumeric characters of the identification feature 200, "CT," are engraved mirror-reversed on the lower surface 151 of the base 16. The "CT" is engraved mirror-reversed so that when imaging technology, such as x-ray imaging, is used to identify a subcutaneously implanted access port, the "CT" will be visible in the proper orientation. By engraving a desired identification feature mirror-reversed on the bottom surface of an access port, a practitioner will be able to determine if there is a problem with the port after implantation, such as if the access port has flipped or otherwise become mis-oriented while in the body of the patient. Thus, if the identification feature is seen mirror-reversed or askew in an x-ray image, the practitioner can correct the problem before attempts are made to use the access port.

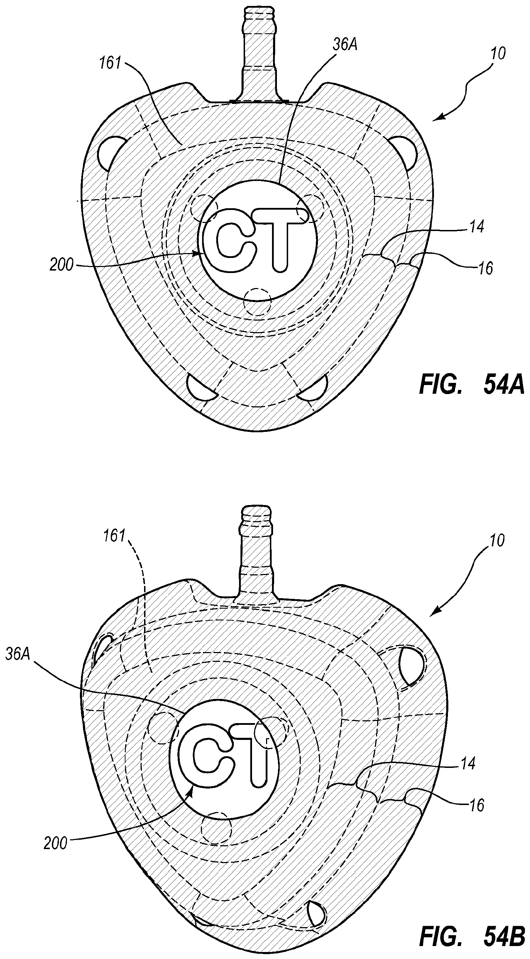

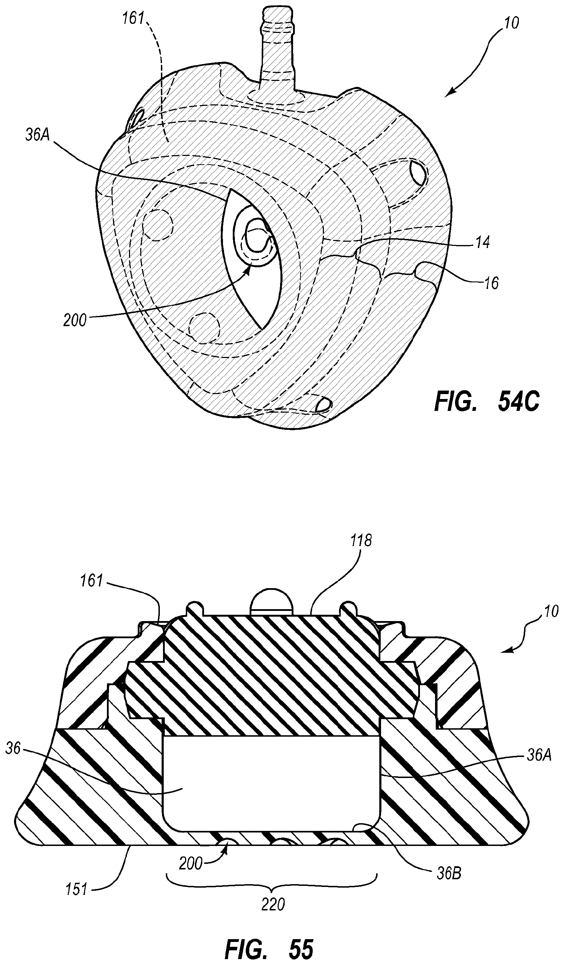

Although also useful in access ports where only a portion of a port includes a metallic material, *e.g*., a metal plate, the engraving technique is well-suited in one embodiment for access ports that are composed of solid metal, such as titanium, stainless steel, or other materials that are typically radiopaque, *i.e*., non-transmissive to x-rays in sufficient thickness. FIGS. 54A-54C are representative images of the access port 10 of FIG. 52, which includes titanium or other metallic material, as seen via x-ray imaging after implantation into the patient. The access port 10 includes the identification feature 200 as seen in FIGS. 52 and 53B. Due to the relative thickness of the access port 10, the material of the base 16 and cap 14 surrounding a cavity periphery 36A of the cavity 36, which is a fluid cavity, is substantially non-transmissive to x-rays and therefore appears relatively dark in the x-ray image of FIG. 54A. However, the material of the access port 10 within the cavity periphery 36A is relatively thinner through a cavity base 220 (as seen in FIG. 55) than through the material of the cap 14 and base 16. Thus, additional thinning of the material when creating the identification feature 200 enables the identification feature to appear relatively more radiographically transmissive than the surrounding material of the cavity base under x-ray imaging. Note that the identification feature 200 in FIG. 54A is visible in the proper orientation, indicating that the access port is not flipped.

FIGS. 54B and 54C are additional representative x-ray images of the identification feature 200 of the access port 10, wherein the access port is tilted at angles of approximately 20 and 50 degrees, respectively. Thus, the identification feature 200 is also useful for determining relative orientation of the access port 10 after implantation.

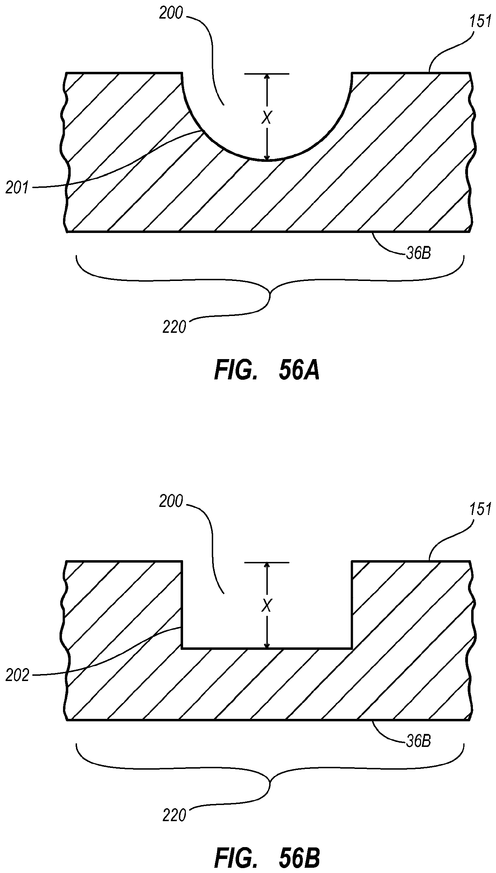

FIG. 55 shows a cross-sectional view taken at line 55-55 of the access port 10 in FIG. 52. In this example embodiment, the identification feature 200 is disposed beneath the septum 118 and the cavity 36. FIGS. 56A and 56B further depict enlarged cross-sectional views of potential cut profiles of the recessed identification feature 200. FIG. 56A shows a rounded engraving profile 201, engraved on the lower surface 151 of the base 16 and used for purposes of aesthetics and ease of manufacturing. For a relatively more defined contrast under imaging technology, however, a sharp-edged engraving profile 202 may be used, as seen in FIG. 56B. Note that a variety of cross-sectional recessed profiles may be employed. This disclosure further contemplates that although engraving is discussed here, other methods of marking the identification feature may be used, such as milling, machining, chemical or laser etching, molding, stamping, etc.

Regardless of the cut profile used, better contrast is achieved generally with greater engraving depth X. The optimal engraving depth X will depend, however, on the thickness of the overall cavity base 220, which is the portion of the base directly below the cavity 36, as shown in FIG. 55. For example, in an embodiment of an access port including titanium, if the overall thickness of the cavity base 220 is approximately 0.020" then sufficient contrast for x-ray imaging purposes can be obtained in one embodiment by engraving the identification feature 200 to a depth X (FIGS. 56A, 56B) of between about 0.009" and about 0.011". In another example embodiment of an access port including titanium, where the overall thickness of the cavity base 220 is approximately 0.030", sufficient contrast can be obtained by engraving the identification feature 200 to a depth X of between about 0.015" and about 0.021". One of ordinary skill in the art will appreciate that the depth of an engraved identification feature can be varied substantially in order to comply with a product's safety requirements and still remain within the scope contemplated by this disclosure. In addition, the depth X of the identification feature can vary according to the position of the feature on the access port, the thickness of material to be penetrated by the imaging technology, the type of material included in the access port, etc.

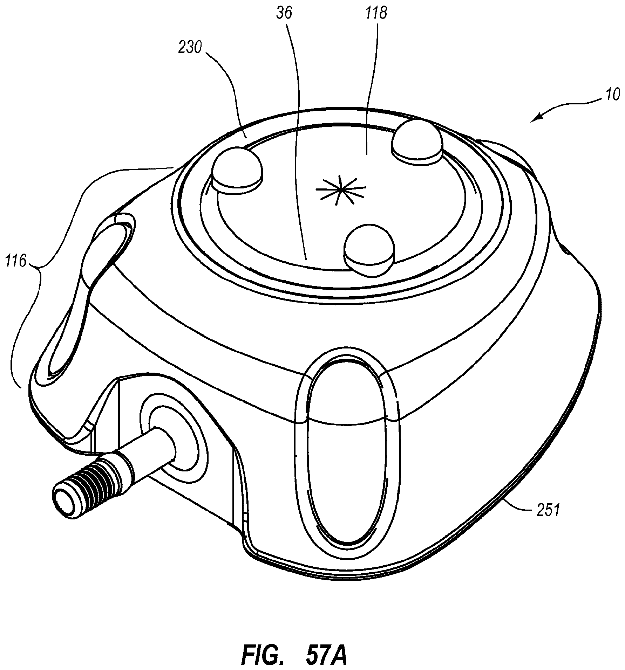

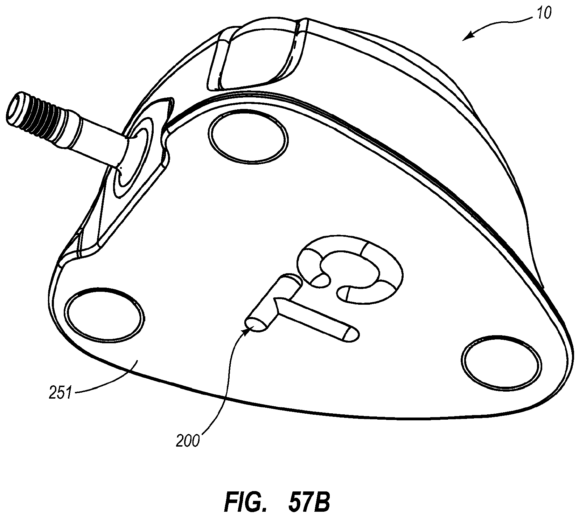

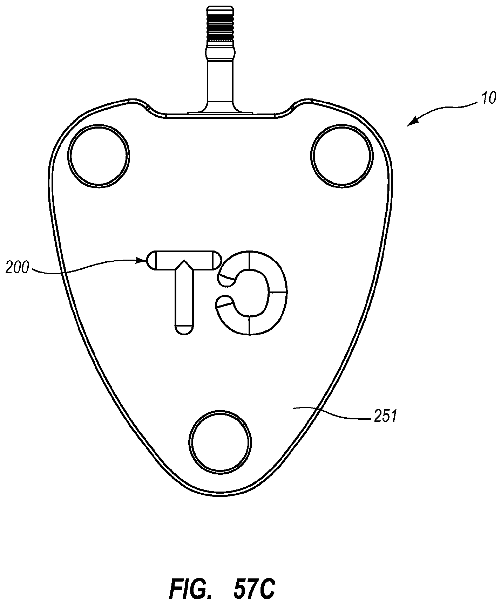

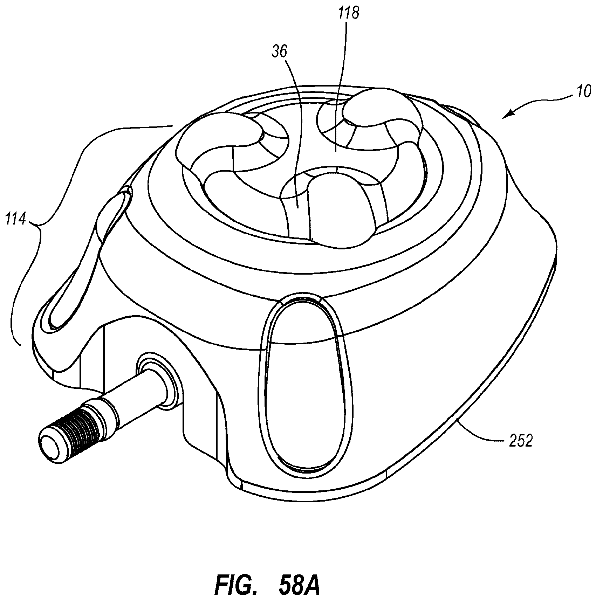

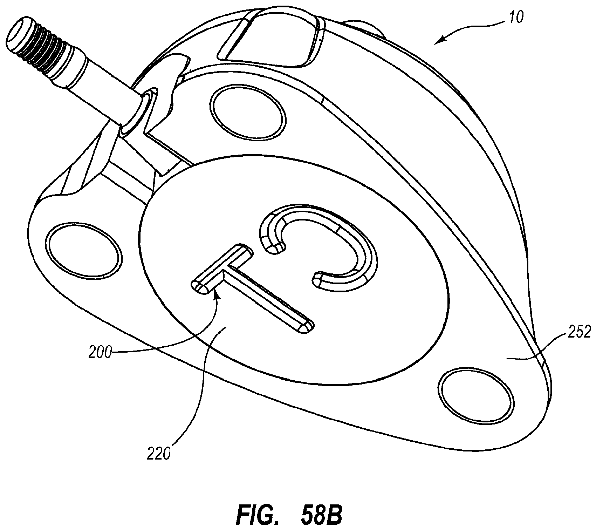

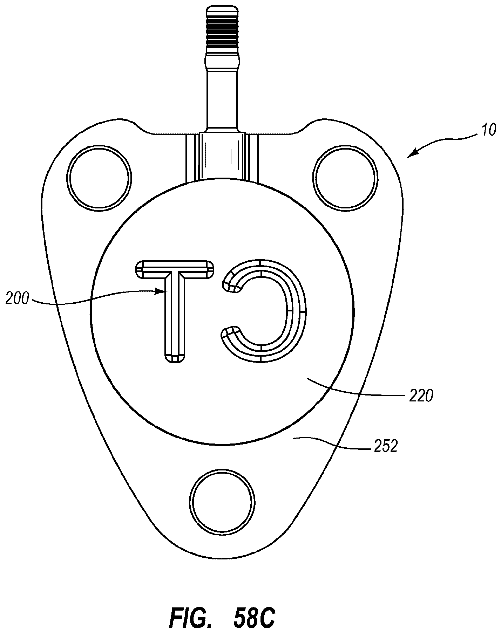

It is also contemplated by this disclosure that the use of an identification feature in a metallic or other radiopaque access port can be applied to access ports having a variety of possible configurations, such as is seen in FIGS. 57A-58C, for example. FIGS. 57A-57C depict one embodiment, wherein the access port 10 includes an identification feature 200 on a lower surface 251 of a base or body 116. The access port 10 in FIGS. 57A-57C includes a retaining ring 230, which seals the septum 118 to the base or body 116, over the cavity 36. In one embodiment, the retaining ring 230 is press fit into the base or body 116 to hold the septum 118 in place. FIGS. 58A-58C show yet another embodiment, wherein the access port 10 includes an identification feature 200 on the cavity base 220 and wherein the cavity base is mated to and flush with a lower surface 252 of a cap 114 to define a body. In a particular embodiment, the cavity base 220 is press fit into the cap 114, though other mating configurations can also be employed.

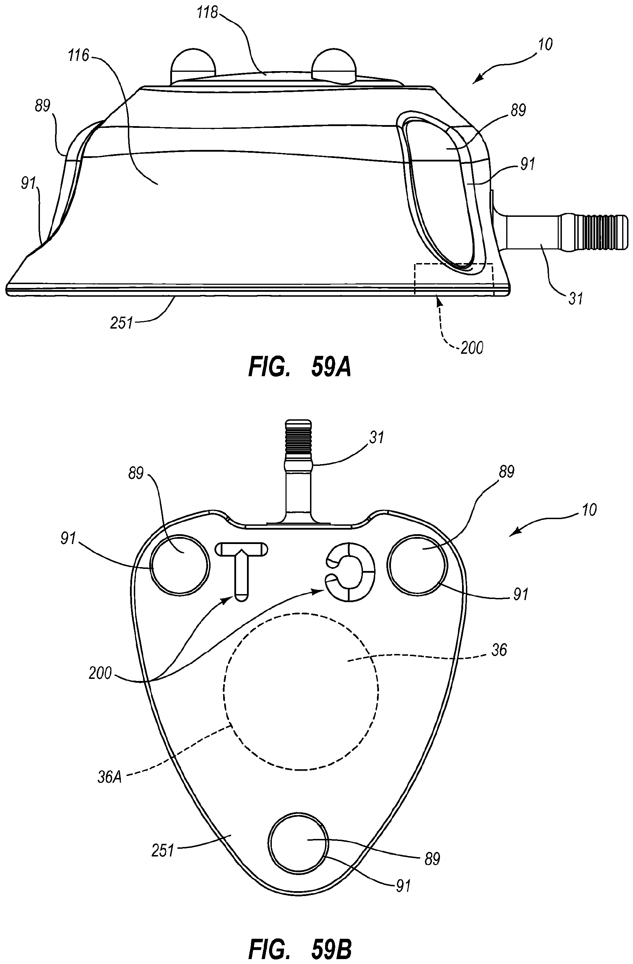

In another embodiment contemplated by the instant disclosure, FIGS. 59A and 59B show that the location of the identification feature 200 can vary as well. Rather than placing the identification feature 200 under the cavity 36, it is possible to place the identification feature under another portion of the access port 10, such as under the outlet stem 31 and between the septum plugs 89, *i.e*., proximate the outer periphery of the access port bottom surface. Though the overall thickness of the access port structure above the identification feature 200 is greater in this location than if engraved under the cavity 36, the change in location allows for a relatively deeper engraving, which will increase contrast without risk of excessive thinning of the cavity base 220. Additionally, in one embodiment, it is possible to define the identification feature compositely by engraving into both the bottom and top surfaces, such that the engravings are vertically aligned. This enables the remaining material thickness to be substantially reduced in order to provide relatively greater radiographic transmission through the identification feature.

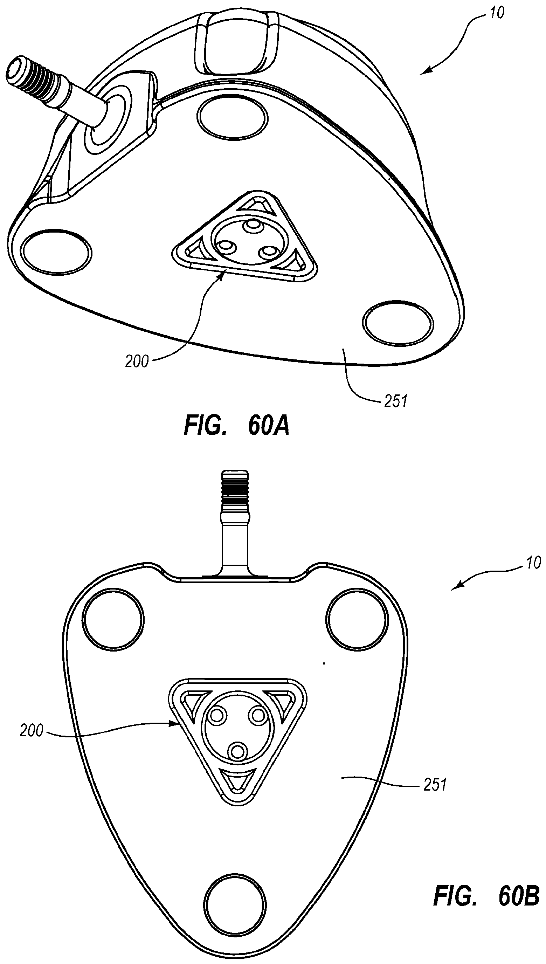

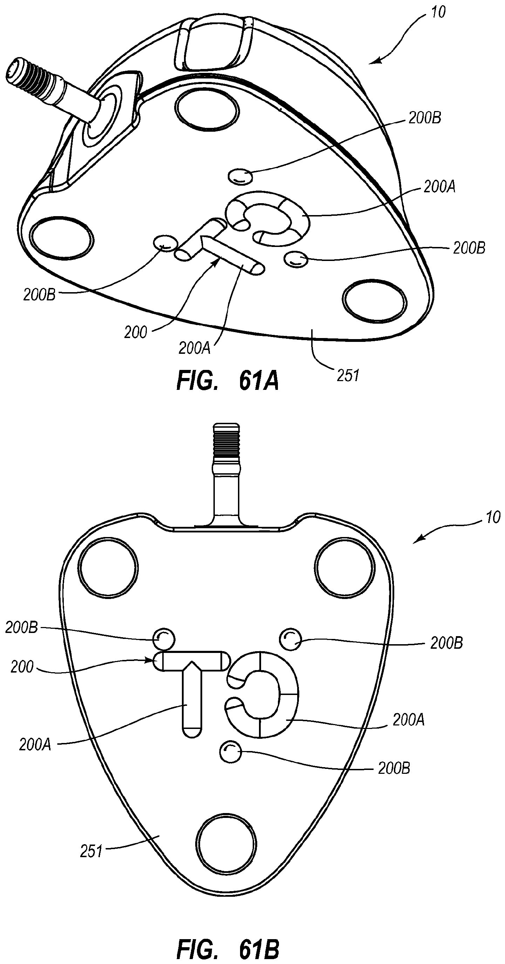

Additionally, the instant disclosure contemplates access ports having any variety or combination of desired identification features for indicating power-injectability or other aspect or characteristic of an access port. Specifically, FIGS. 60A-61B depict different types of identification features 200, according to example embodiments. FIGS. 60A-60B depict a symbolic identification feature 200. FIGS. 61A-61B depict an exemplary embodiment of an access port 10 including a combination of identification features 200, namely an alphanumeric identification feature 200A and a patterned identification feature 200B. A patterned or symbolic identification feature can also be used to help indicate the orientation of the port or for any other desired reason. It is understood by the instant disclosure that other symbols, patterns, marks, and alphanumeric characters can be used both alone and in any combination with each other on a variety of access port configurations.

In additional embodiments, the identification feature can be defined on an inside bottom surface 36B of the cavity 36 of an access port 10, or in addition to the identification feature 200 provided on the bottom surface 251. In another embodiment, the material surrounding the defining edges of the desired radiopaque alphanumeric character, symbol, pattern, etc., can be removed instead of removing the desired feature shape itself so as to define a "positive" relief image of the identification feature. Such a positive relief identification feature can be defined on a lower surface of an access port body or on the inside bottom surface of the cavity, for example.

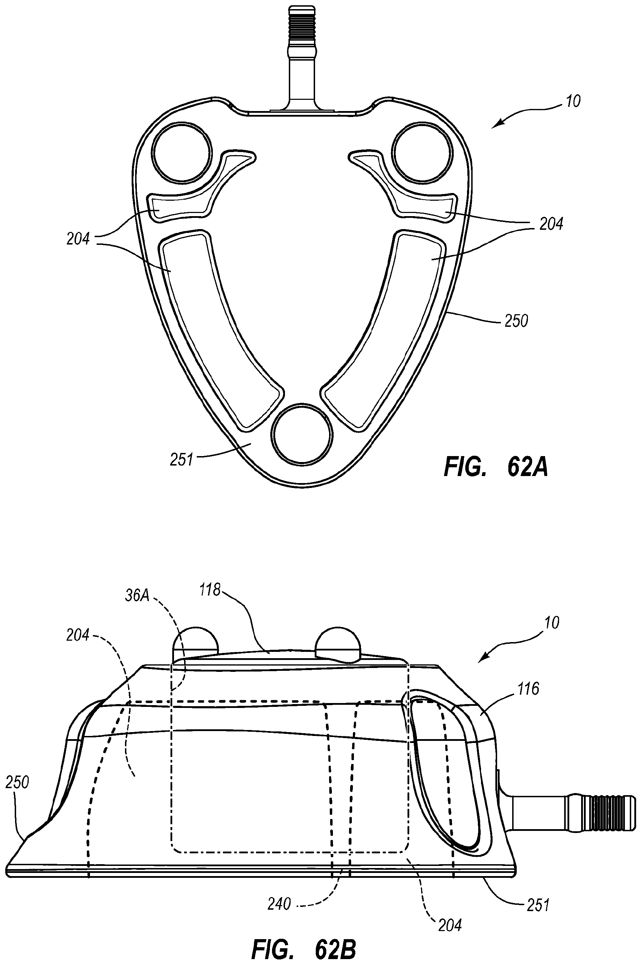

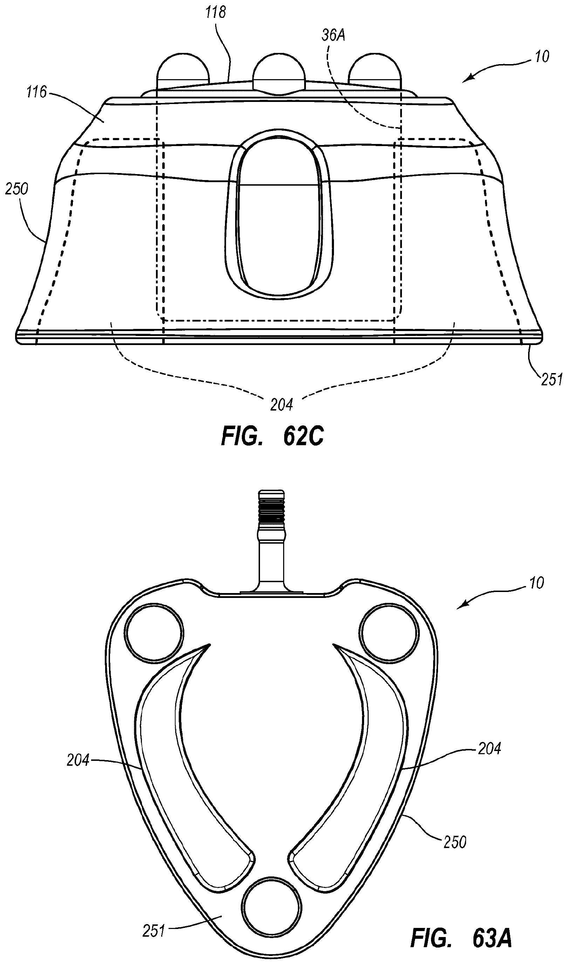

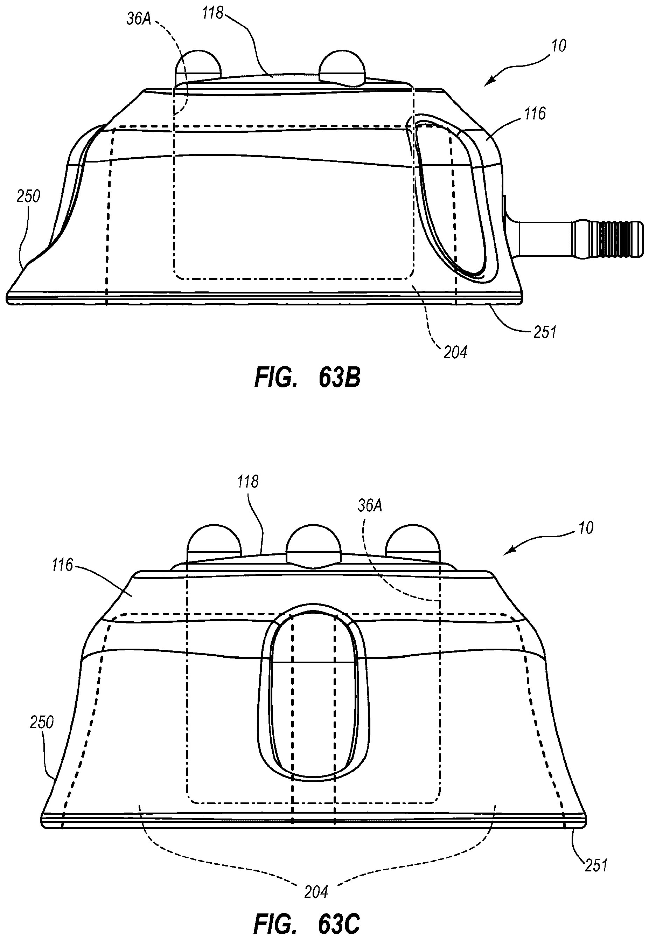

In addition to the various types of symbols, patterns, marks, and alphanumeric characters that are contemplated by the instant disclosure, FIGS. 62A-63C disclose additional example embodiments of identifying features on access ports that are observable via x-ray or other suitable imaging technology. Specifically, the instant disclosure contemplates the use of shelled-out cavities 204, wherein portions of the access port 10 are hollowed out. This results in shelled-out cavities 204 extending inward from the lower surface 251 of the base or body 116 or corresponding port lower surfaces of the other embodiments described herein, including the lower surface 151 of the base 16, as in FIG. 151, and the lower surface 252 of a cap 114, as in FIGS. 58A-58C. This is done by removing the material surrounding the cavity 36 without disrupting the cavity periphery 36A or the outer side surfaces 250 of the access port 10. As seen in FIG. 62B, ribs 240 may be left to support the remaining "shelled" frame of the access port 10. The definition of such cavities 204 provides a relative difference in radiopacity of the access port 10 that can be identified via x-ray imaging. As such, the cavities 204 can be arranged to define a pattern or to form an indicia for identification of an aspect or characteristic of the access port 10. Note that in other embodiments, the cavities can be defined so as to extend from other surfaces of the access port, including the top and sides thereof.

In a further aspect contemplated by the instant disclosure, it is contemplated that a communicative technology may be utilized wherein information is encompassed by an access port contemplated by the instant disclosure. Generally, a communication device (e.g., a radio beacon, a light-emitting element, an ultrasound emitting transducer, etc.), may be imbedded or otherwise affixed to an access port contemplated by the instant disclosure. Such a communication device may be configured for transmitting information in response to a given impetus. More specifically, the instant disclosure contemplates that an access port contemplated by the instant disclosure may be exposed to a request signal (e.g., a sound, an impact or an acceleration, light, radio waves, etc.). Such a request signal may cause the communication device to transmit information therefrom via sound, light, radio waves, or as otherwise known in the art. Such information may be employed for identifying an access port contemplated by the instant disclosure.

In one exemplary example, it is contemplated that radio frequency identification technology may be employed for identification of an access port contemplated by the instant disclosure. Particularly, so-called active RFID tags are powered by an internal battery and are typically read/write devices. Currently, a suitable cell coupled to suitable low power circuitry can ensure functionality for as long as ten or more years, depending upon the operating temperatures and read/write cycles and usage. So-called passive RFID tags operate without a separate external power source and obtain operating power generated from the reader. Passive RFID tags are typically programmed with a unique set of data (usually 32 to 128 bits) that cannot be modified. Read-only tags may operate as an identifier comparable to linear barcodes which may contain selected product-specific information. Thus, passive RFID tags may be much lighter than active RFID tags, less expensive, and may offer a virtually unlimited operational lifetime. The tradeoff is that they have shorter read ranges than active tags and require a higher-powered reader.

One advantage of RFID approach is the noncontact, non-line-of-sight nature of the technology. Tags can be read through a variety of substances such as snow, fog, ice, paint, crusted grime, and other visually and environmentally challenging conditions, where other optically read technologies may be less effective. RFID tags can also be read in challenging circumstances at rapid speeds, in most cases responding in less than about 100 milliseconds.

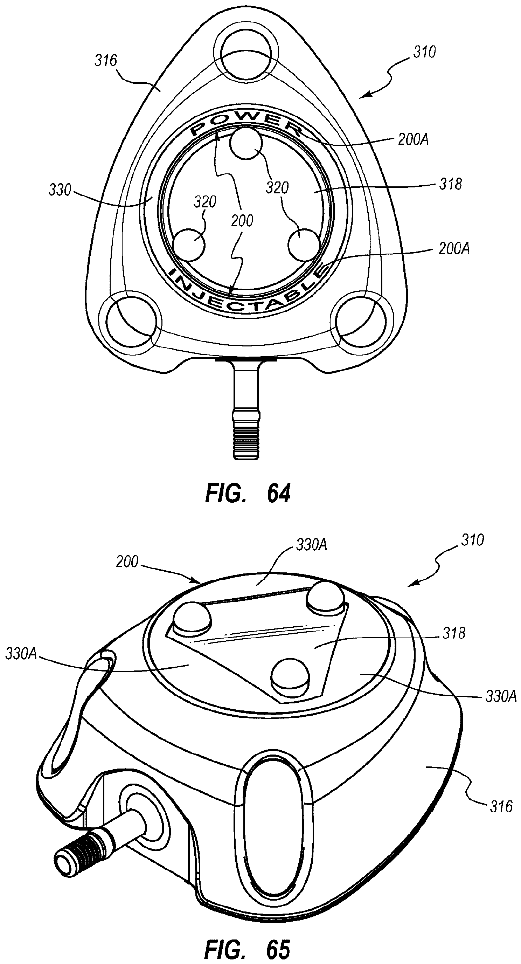

Reference is now generally made to FIGS. 64-75C in describing additional embodiments wherein an access port includes at least one identification feature observable through interaction with an imaging technology, such as x-ray and fluoroscopy, for instance, in order to facilitate identification of at least one attribute, or characteristic, of an access port subsequent to implantation within the body of a patient. It is appreciated that the embodiments to be described can be included alone or together with other identification features described herein and may be employed with access ports having a variety of sizes, shapes, and other variations in configuration. As such, the embodiments described herein are merely examples of the principles of the present disclosure.

FIG. 64 shows an access port 310 including a base 316 and a septum 318 covering a reservoir defined by the base. The septum 318 includes a plurality of palpation bumps 320 for enabling external digital palpation and location of the septum by a clinician after the access port 310 has been subcutaneously implanted. The port 310 includes a retaining ring 330 for capturing and retaining the septum 318 in place atop the port reservoir. In the present embodiment, both the port base 316 and the retaining ring are metallic substance, including titanium for instance, though in other embodiments other suitable materials may be used.

In the present embodiment the retaining ring 330 includes an identification feature 200 for identifying a predetermined attribute or characteristic of the port 310 after implantation thereof. Specifically, the retaining ring 330 includes alphanumeric character identification features 200A spelling "POWER INJECTABLE," which indicates that the port 310 is capable of power injection. The alphanumeric characters in one embodiment are inset via etching or otherwise suitably defined in the retaining ring 330 so as to provide a relative thickness difference between the characters and surrounding metallic retaining ring material, thus providing a corresponding radiographic contrast when the port 310 is imaged with x-ray imaging technology. This contrast enables the alphanumeric characters to become visible in an x-ray and therefore discernible by a clinician viewing the x-ray, thus enabling the port attribute or characteristic relating to the identification feature 200 to be ascertained.

Note that the alphanumeric identification features 200A can be defined on the retaining ring 330 in any number of suitable ways, including etching, engraving, etc., and the characters can be defined partially or completely through the retaining ring. Also, the particular characters or words used can vary from what is described here. Indeed, other characters, patterns, symbols, etc. can be employed in the identification feature 200. Optionally, the identification features can be defined in negative relief, as shown in FIG. 64, or in positive relief, if desired.

Additionally, in other embodiments the identification feature of the retaining ring can be configured in other ways according to the configuration of the port. For instance, in embodiments where the port body includes a non-metallic material, the identification feature can include radiopaque ink that is applied to a surface of the retaining ring so as to form the alphanumeric or other characters or features. In yet other embodiments, the identification feature can be included on portions or surfaces of the port in addition to the retaining ring. These and other modifications are therefore contemplated.

FIG. 65 includes the metallic retaining ring 330 of the metallic port 310 configured in accordance with another embodiment, wherein the retaining ring defines the identification feature 200, including a plurality of overlapping portions 330A that each overlap a portion of the septum 318 retained by the retaining ring. In FIG. 65, the overlapping portions 330A of the retaining ring 330 cooperate to generally define a triangular shape, which provides a radiographic contrast relative to portions of the metallic port 310 below the retaining ring. As before, this provides a corresponding radiographic contrast when the port 310 is imaged with x-ray imaging technology, enabling the triangular shape to be discernible as a radiopaque outline by a clinician viewing the x-ray in order to ascertain the predetermined port attribute or characteristic relating to the identification feature 200 to be ascertained. In other embodiments, the retaining ring can define other shapes in addition to the triangular shape shown here. Additionally, characters, symbols, or other patterns can be defined in or included on the overlapping portions of the retaining ring if desired.

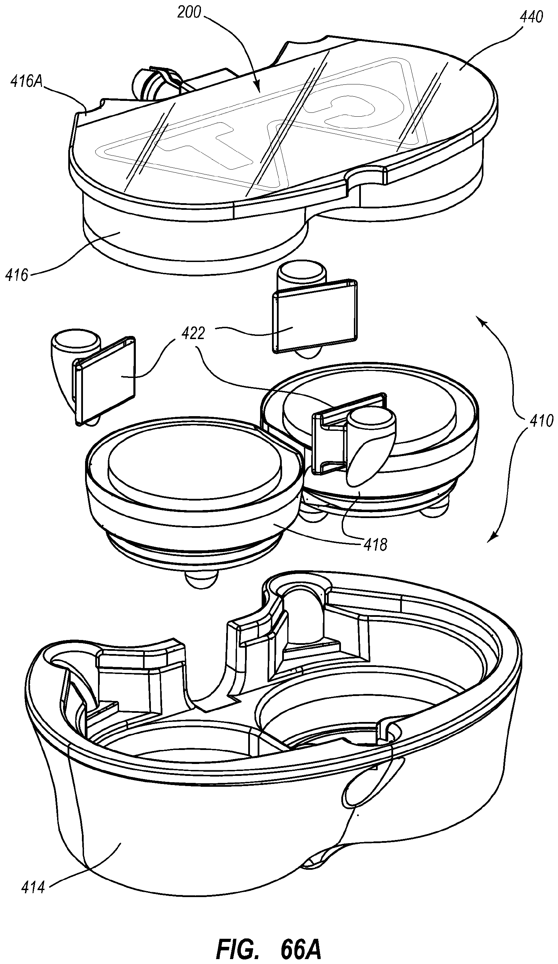

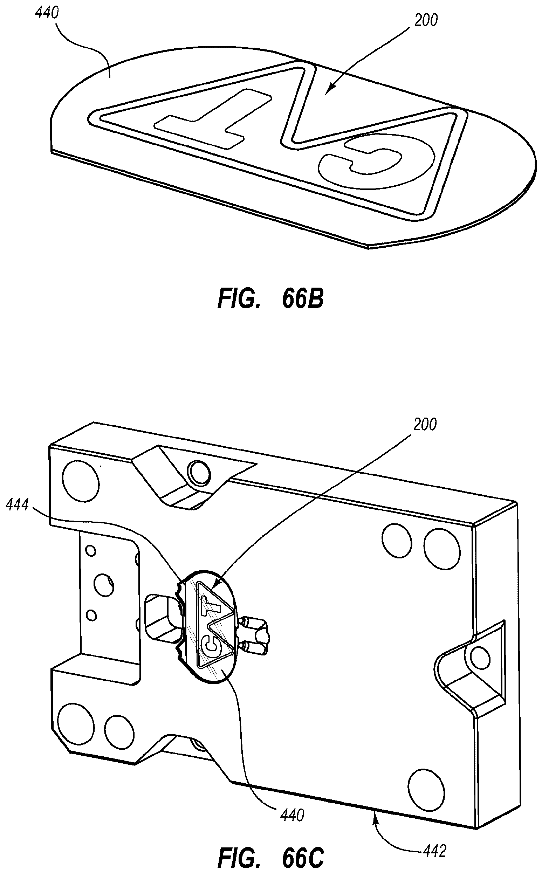

FIGS. 66A-66D depict various details regarding the inclusion of an identification feature for identifying a predetermined attribute or characteristic of an access port after implantation into a patient. Specifically, these figures depict a dual reservoir access port 410, including a cap 414 matable to a base 416 and two septa 418 interposed between the cap and base. Suture plugs 422 are included with the port 410. In accordance with the present embodiment, a bottom surface 416A of the port base 416 includes the identification feature 200 for identification of the subcutaneously implanted port. As best seen in FIG. 66B, the identification feature 200 in the present embodiment includes a radiopaque marking including the letters "C" and "T" outlined by a double-triangle border, though many different character, pattern, and/or combination configurations are possible. For instance, in addition to identifying the access port as power injectable, this and other identification features described herein can be used to designate lot numbers, hospital identification, port brand, etc.

The radiopaque marking of the identification feature 200 can include a metallic powder intermixed with an ink-based marking. Specifically, in one embodiment, the radiopaque marking includes tungsten powder intermixed with 1020 black wire marking ink manufactured by Gem Gravure, Inc. of West Hanover, MA, in a ratio of three parts tungsten powder to one part ink. Mixing of the two components can include ball mixing to ensure good component integration in one embodiment. Also, additives can be added to the mixture to attain a proper mixture viscosity.

In other embodiments, the powder-to-ink ratio can be modified from that described above, including 2:1, 4:1, and 5:1 ratios, for instance. The ideal ratio will vary according to the type of materials employed in the mixture, the density of the desired image, powder particle size, amount of mixture applied to the port, etc. In yet other embodiments, other medical grade inks or suitable liquids, as well as other biocompatible metallic powders or suitable radiopaque materials, could be used. In one embodiment, a ceramic, such as zirconium oxide powder, can be intermixed with a marking ink to provide the radiopaque marking. Ink thinners can also be added to the mixture, along with other suitable substances as appreciated by those skilled in the art.

As shown in FIG. 66B, the ink-based radiopaque marking that forms the identification feature 200 in the present embodiment is included on a substrate 440. In one embodiment, the substrate 440 includes a material substantially identical to the material included in the port 410. Specifically, in one embodiment, both the port 410 and the substrate 440 include an acetyl resin sold under the brand DELRIN^{®} by E. I. du Pont Nemours and Company, though it is appreciated that other suitable materials could be used for the substrate and port.

The substrate 440 is employed as a base on which the radiopaque marking can be deposited in preparation for integration of the substrate and marking into the port 410 during an injection molding process so as to encapsulate the radiopaque marking within the molded port. In detail, in one embodiment, the radiopaque marking, including the above-described ink/powder mixture or other suitable substance, is first deposited on a surface of the substrate 440 via any acceptable process, including pad printing, manual or automatic painting, silk screening, use of a template, etc. To improve adhesion of the ink/powder mixture, the substrate can be plasma treated or corona treated in one embodiment.

Once the radiopaque marking has been applied to the substrate 440, the substrate is loaded into a mold, such as that shown in FIG. 66C, which depicts the substrate positioned within a cavity 444 of a portion of a mold 442. The substrate 440 is positioned within the mold cavity 446 such that the radiopaque marking is facing in toward what will become the center of the port 410. In one embodiment, the substrate 440 is held in place within the mold cavity 444 via a vacuum assist system; in other embodiments, temporary mechanical fixation can be employed, if necessary. A template including a hole sized to enable the substrate to pass therethrough can be used in one embodiment to assist the technician in placing the substrate 440 with the proper orientation within the mold cavity 444.

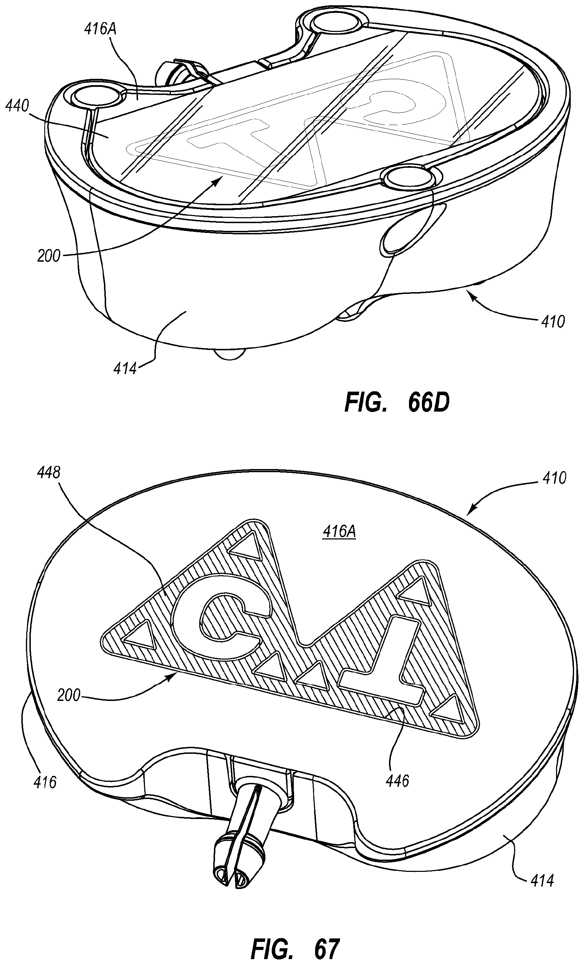

The port 410 is then fabricated by an injection molding process. The substrate 440 is thus insert-molded into the port 410 via the injection molding process, which bonds the substrate 440 to the molded body of the port 410, thus encapsulating the radiopaque marking of the identification feature 200 within the port and preventing its inadvertent removal. Additionally, due to the relative thinness of the substrate 440, the identification feature remains visible through the substrate from outside of the port 410, as seen in FIG. 66D, before implantation. In one embodiment, the thickness of the substrate 440 ranges from about 0.002 inch to about 0.015 inch, though other thicknesses can be acceptably used. Later, when the port 410 is implanted and imaged under x-ray, the identification feature 200 will be visible in the x-ray image and useful to identify an attribute or characteristic of the implanted port.

It is appreciated that in other embodiments, the substrate can be configured to be positioned in other regions of the port. In yet other embodiments, other substrate materials can be used. For instance, in one embodiment the substrate can include woven high-density polyethylene sold under the brand TYVEK^{®}. In this case, the substrate 440 does not permanently adhere to the port 410 as a result of the insert molding process, but is removed after molding process is complete. The radiopaque marking ink/powder mixture initially included on the woven substrate 440, however, is integrated into the port body and remains with the port 410 after molding and substrate removal to serve as the identification feature 200. Flaps or flanges can be included on the substrate to facilitate its separation from the substrate from the port after molding, in one embodiment. In another embodiment, the ink/powder radiopaque marker mixture is allowed to dry on the substrate 440 after application thereon to improve adhesion to the port 410 during the insert molding process. In addition to those explicitly described here, other suitable materials can be used as the substrate. In yet another embodiment, no substrate is used and the ink/powder radiopaque marker mixture is applied directly to the mold surface before the port 410 is molded therein.

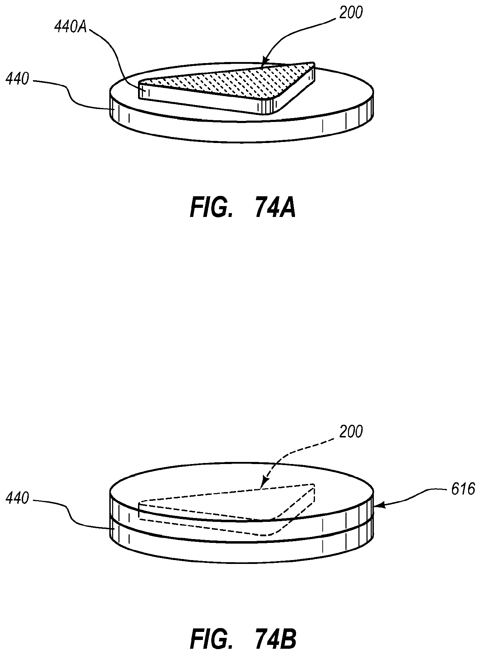

FIGS. 74A and 74B depict details of the substrate 440 and identification feature 200 configured in accordance with another embodiment, wherein the substrate forms a portion of the port base. A raised surface 440A is included on the substrate, and a radiopaque marking, such as the intermixed marking ink and radiopaque powder, is included on the raised surface to define the identification feature 200. Application of the radiopaque marking can occur in any one of a number of suitable ways, including contact application by a stamp or tamp pad, ink jet printing, physical or chemical deposition, etc.

The substrate 440 with the included identification feature 200 can then be inserted into a mold and insert-molded to form part of a base 616 of an access port. The radiopaque identification feature 200, now encapsulated within the base, provides the desired identification of a predetermined attribute or characteristic of the port once manufacture of the port is complete.

Reference is now made to FIG. 67, which depicts another identification feature for an access port, such as a plastic port for instance, according to one embodiment. In particular, the port 410 of FIG. 67 includes a cavity 446 defined on a bottom surface 416A of the port base 416. In one embodiment, the cavity 446 is defined to a depth of about 0.010 inch, though other depths can also be used according to desire and port configuration. The cavity 446 is filled with a radiopaque fill material 448. The cavity 446 is shaped with a predetermined design or configuration so as to form the identification feature 200 when filled with the radiopaque fill material 448, thus enabling a predetermined attribute or characteristic of the port 410 to be identified via x-ray imaging subsequent to implantation. In the present embodiment, the fill material 448 includes tungsten powder intermixed with a two-part silicone sold under the brand SILASTIC^{®} Q7-4840, available from Dow Corning Corporation, Midland, MI in equal quantities, *i.e*., equal parts of part A silicone, part B silicone, and tungsten powder. Of course, other suitable materials could also be employed. For instance, titanium can be used in place of tungsten, and biocompatible urethane adhesives can be used in place of silicone.

In one embodiment, the fill material 448 is injected into the cavity 446 by a pressurized syringe, such as an electronic fluid dispenser, though other suitable techniques can also be employed, including manual filling by syringe. Any excess fill material 448 can be removed from the port base bottom surface 416A after filling, and the fill material can be allowed to cure. Note that in other embodiments the bottom surface of the port can include other portions of the port in addition or instead of the base, as shown in FIG. 67.

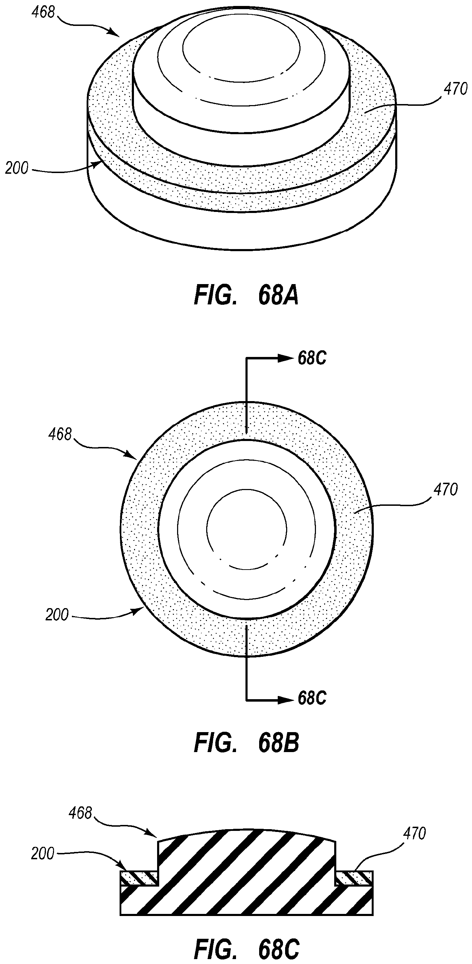

FIGS. 68A-68C show details of one embodiment for providing the identification feature 200 on a resilient septum 468 of an implantable access port, such as a plastic port for instance, wherein the septum includes a radiopaque portion visible under x-ray imaging to provide information relating to an attribute or characteristic of the septum itself and/or the access port in which the septum is disposed. In the illustrated embodiment, the radiopaque portion is defined as an annular portion 470 disposed about the upper outer periphery of the septum 468 so as not to interfere with puncturing of the septum by needles during port use. As best seen in FIG. 68C, the annular portion does not extend in depth through the thickness of the septum outer portion, but in other embodiments the thickness, size, and position of the radiopaque portion can vary on the septum.

In the present embodiment, the radiopaque annular portion 470 includes barium sulfate-loaded silicone, while the remainder of the septum 468 is unloaded silicone. In other embodiments, other suitable radiopaque materials can be employed with silicone or other septum materials. In one embodiment, the septum 468 of FIGS. 68A-68C can be formed by a two-part molding process, wherein the annular portion 470 is manufactured separately from the rest of the septum 468, then the two parts are adhered together by a suitable adhesive, mechanical fixation, etc., to form the structure shown in FIGS. 68A-68C.

In another embodiment, the present septum 468 is manufactured integrally via a co-molding process, wherein separate injection heads are employed in a mold cavity in order to injection mold the annular portion 470 with one or more heads and the rest of the septum 468 with separate heads. These and other manufacturing methods are therefore considered within the spirit of the present disclosure.

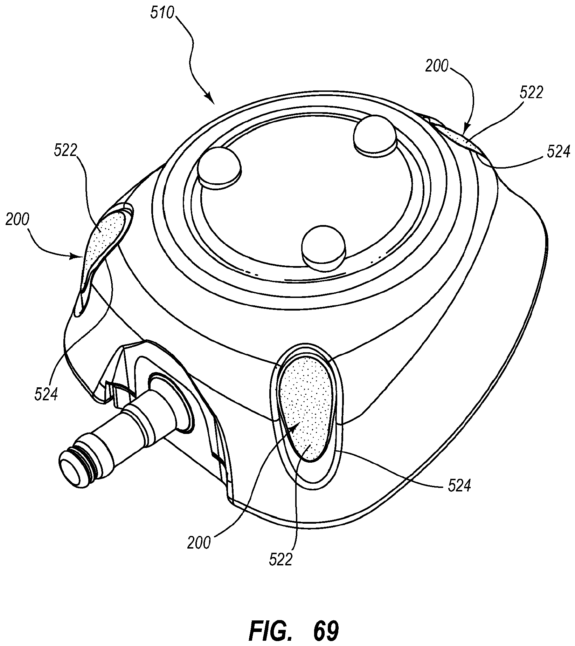

The principles discussed in connection with FIGS. 68A-68C can be expanded in one embodiment shown in FIG. 69, wherein a port 510 including resilient suture plugs 522 disposed in corresponding suture plug holes 524 is configured such that the suture plugs include a radiopaque material, such as the barium sulfate-loaded silicone employed in the septum 468 of FIGS. 68A-68C or other suitable radiopaque material. So configured, the suture plugs provide the identification feature 200 that is visible under x-ray imaging to provide information relating to an attribute or characteristic of the port 510. In one embodiment, the port 510 can include both the radiopaque suture plugs 522 and the septum 468 including the radiopaque portion 470 in order to provide additional identification ability and/or to provide information relating to the orientation of the port within the body of the patient. In addition to barium sulfate, the suture plugs can include tungsten, tantalum, or other suitable radiopaque materials. In yet another embodiment, one or more radiopaque beads can be disposed in the port body to provide similar port visibility under x-ray.

In one embodiment, the septum, suture plugs, or other portion of the port can include an ultraviolet light-sensitive material. The ultraviolet light-sensitive material can be applied to the surface of the port component or can impregnated into the component. After implantation of the port, ultraviolet light is directed through the skin of the patient to be incident on the ultraviolet light-sensitive material of the port, which causes the material to fluoresce with visible light that is observable through the skin of the patient, thus identifying the port and/or its predetermined attribute or characteristic.

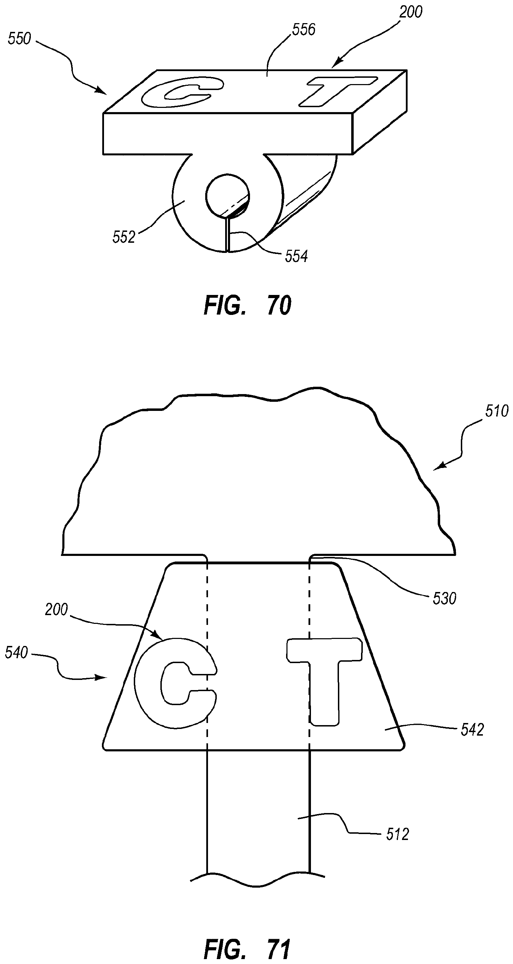

It is appreciated that a radiopaque identification feature can be included or associated with a port in other ways in addition to those embodiments already described. Examples of this can be found in the embodiments depicted in FIGS. 70-72. In FIG. 70, for example, an identifier tag 550 is shown, including a ring portion 552 with a slit 554 for enabling the identifier ring to be attached to a catheter that is operably attached to the stem of a port. The identifier tag 550 further includes a face portion 556 on which a radiopaque identification feature 200 can be placed for visibility via x-ray imaging to identify a predetermined attribute or characteristic of the port. The tag can be designed in various different shapes and configurations. For instance, the tag can be included as part of a catheter securement device for locking an end of a catheter to the stem of the port.

In FIG. 71, the port 510 is shown with a catheter securement device 540 that is used to secure the connection between an end of a catheter 512 and a stem 530 of the port. A body 542 of the catheter securement device 540 is configured to include the identification feature 200 for visibility via x-ray imaging to identify a predetermined attribute or characteristic of the port to which the device is attached. Again, the shape, size, and particular configuration of the catheter securement device and identification feature can vary from what is shown and described herein.

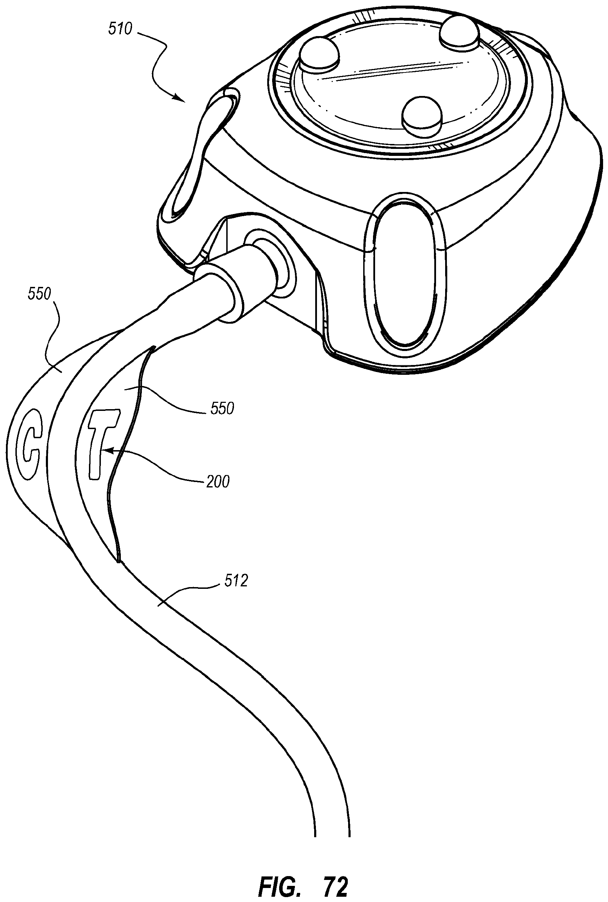

In FIG. 72, the port 510 is shown with the catheter 512 operably attached thereto. The catheter 512 includes two flaps 550 that extend from the body thereof, on which the identification feature 200 is included in order to provide a visible identification of a predetermined attribute or characteristic of the catheter and/or port when imaged under x-ray. Of course, the particular identification feature, as well as the number and size/configuration of the catheter flaps can vary from what is described herein.

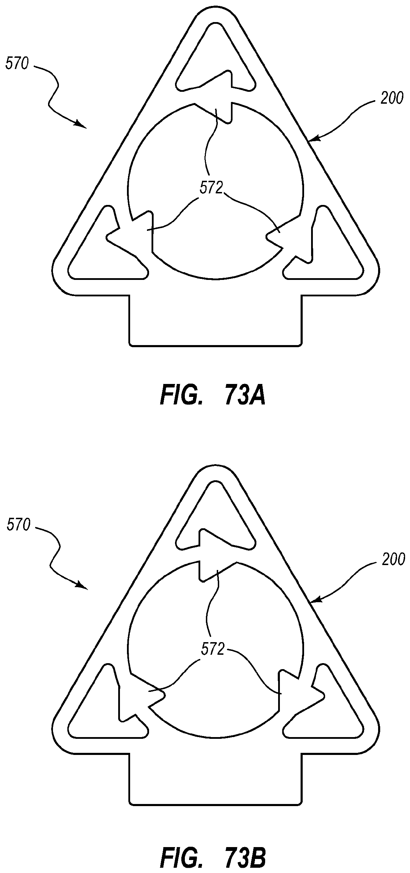

FIGS. 73A and 73B depict yet another example of a radiopaque identification feature wherein the identification feature 200 is included in an insert 570 formed from a radiopaque material, such as tungsten or other suitable material. The insert 570 is suitable for placement in a plastic or other radiotranslucent port such that the insert is visible under x-ray imaging to identify an attribute or characteristic of the port. Orientation arrows 572 provide useful indicia of the orientation of the port. By examining the direction of the arrows 572, a clinician observing an x-ray image of the port insert 570 can determine whether the port is flipped in the body of the patient. In addition to these, other indicia indicating port orientation can be included on the insert in other embodiments.

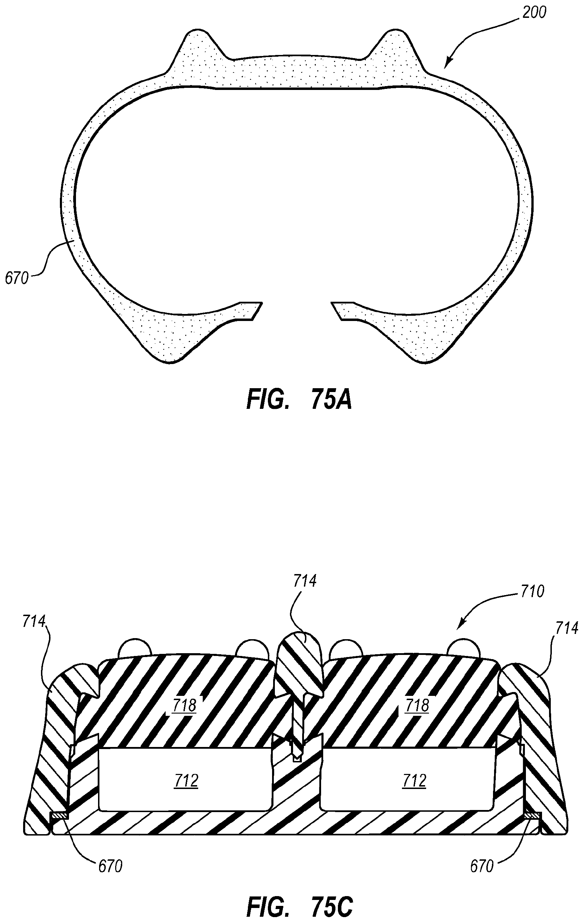

FIGS. 75A-75C show implementation of another example of a radiopaque insert, in addition to that shown in FIG. 73A and 73B, which is included to serve as the identification feature 200 for identifying a predetermined attribute or characteristic of a port, including a plastic port, as in the present embodiment. In particular, a radiopaque insert 670 is shown, configured to be interposed between a cap 714 and a base 716 of a port 710. Note that, though the insert 670 shown here is configured to fit over a dual fluid cavity 712 of the port 710, other inserts including a variety of radiopaque compositions can be configured to be included in other ways with a port. Additionally, the port can define one, two, or more fluid cavities covered by septa 718, without limitation.

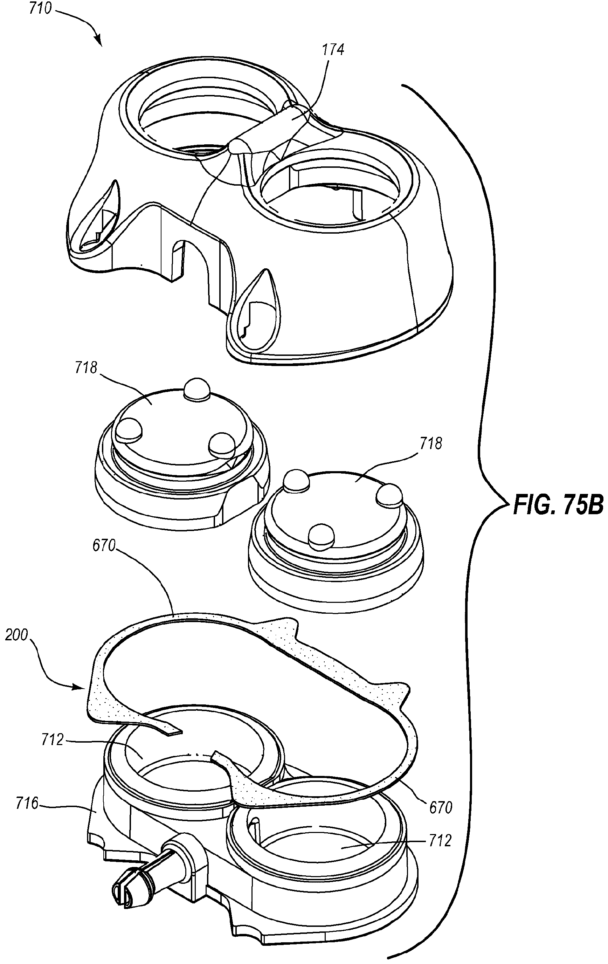

As shown in FIG. 75B, the insert 670 fits over the fluid cavities 712 of the port 710 so as to rest on a portion of the port base 716. So positioned, the insert 670 is sandwiched and secured between the base 716 and the cap 714 when the base and cap are mated together to form the port 710. Such mating can be accomplished by ultrasonic welding, adhesives, etc. The resulting interposition of the insert 670 between the base 716 and cap 714 is shown in FIG. 75C. When the port 710 is later imaged via x-ray after patient implantation, the insert 670 is readily visible, thus enabling the predetermined attribute/characteristic(s) of the port to be identified.

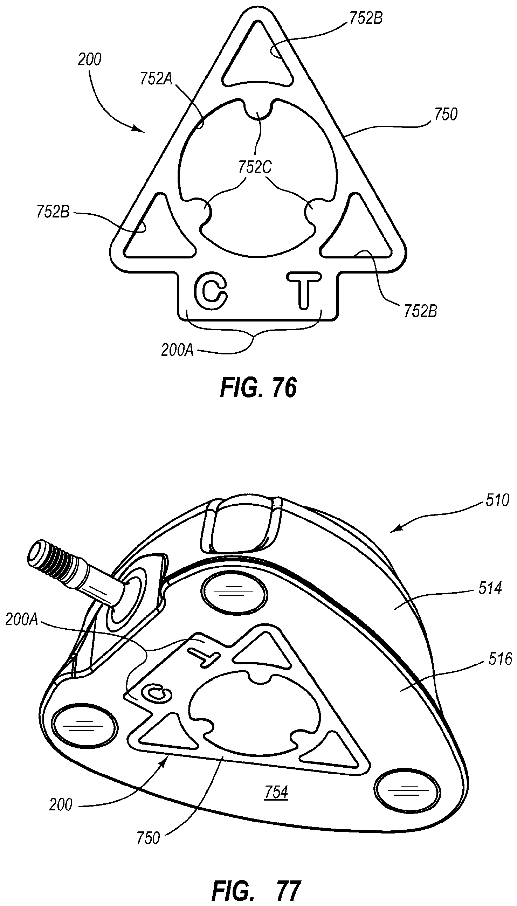

Reference is now generally made to FIGS. 76-77 in describing additional embodiments of an identification feature 200 that is observable through interaction with an imaging technology, such as x-ray and fluoroscopy, for instance, in order to facilitate identification of at least one attribute or characteristic of an access port or other implantable medical device including the identification feature, subsequent to implantation of the device within the body of a patient. It is appreciated that the embodiments to be described can be included alone or together with other identification features described herein and may be employed with access ports having a variety of sizes, shapes, and other variations in configuration. As such, the embodiments described herein are merely examples of the principles of the present disclosure.

In particular, FIG. 76 shows a radiopaque insert 750 including the radiopaque identification feature 200. The insert 750 generally defines a triangular shape and encompasses a central circular hole 752A and three triangular holes 752B disposed near the vertices of the triangular shaped insert. Three inward extending bumps 752B are included about the periphery of the central circular hole 752A.

Alphanumeric indicia 200A are also included on a lower portion of the insert 750, though it is appreciated that such indicia can vary in positional placement, size, type, etc. The indicia 200A of the identification feature 200 in the present embodiment include the letters "C" and "T" and indicate an attribute of the access port in which the insert is included, such as the access port 510 shown in FIG. 77.

In detail, FIG. 77 shows the insert 750 disposed on a bottom surface 752 of a base portion 516 of the access port 510, though other positional relationships of the insert and the access port are possible. The insert 750 is positioned such that the alphanumeric indicia 200A are in reverse configuration when the insert 750 is viewed from the bottom of the access port 510, such as the view shown in FIG. 77. In this way, the alphanumeric indicia 200A are visible through the access port 510 in a forward configuration when the port is imaged from above by x-ray technology.

As already indicated, the indicia 200A of the identification feature 200 in the present embodiment include the "C" and "T" letter-shaped holes that are defined through the insert 750 and indicate a predetermined attribute of the access port 510. In the present embodiment, the identification feature 200 and the alphanumeric indicia 200A indicate that the access port 510 is capable of power injection. Of course, other attributes of the access port can be designated by the identification feature, if desired.

The insert 750 is configured to be radiopaque so as to provide the identification feature 200 when the access port 510 or other suitable medical device that is not sufficiently radiopaque is imaged under x-ray. Examples of access ports not sufficiently radiopaque to be suitably imaged include those including a thermoplastic, such as acetyl resin for instance. When so imaged, the insert 750 of the access port 510 is visible in the radiographic image and will therefore provide desired identification to a clinician viewing the x-ray image of the predetermined port attribute relating to the identification feature 200. In particular, the radiopacity of the insert 750 itself provides a contrast to the radiotranslucent "C" and "T" alphanumeric indicia 200A and other features that are defined through the insert, thus enabling those features to be readily identified in an x-ray image.

It is appreciated that the particular items employed in the identification feature and indicia can vary from what is described here. Indeed, a variety of characters, symbols, patterns, words, etc. can be employed. Optionally, the identification features can be defined in negative or positive relief, as desired. Further, it is appreciated that the geometric holes and indicia described above in connection with the identification feature 200 of the insert 750 can define together or separately one or more attributes of the access port 510 or other implantable device including the insert, as may be appreciated by one skilled in the art. Of course, the shape of the insert itself can also vary from what is shown here.

In the present embodiment, the insert 750 is composed of a mixture including acetyl resin and bismuth trioxide. In one embodiment, for instance, the insert 750 is composed of a mixture including about 70 percent by weight acetyl resin, *e.g*., polyoxymethylene ("POM"), sold under the brand DELRIN^{®} and about 30 percent bismuth trioxide by weight. Other relatively differing concentrations of these two materials can also be used, depending on the desired radiopacity of the insert and other factors. For instance, relatively smaller or greater concentrations of bismuth trioxide may be employed, including 10, 20, 50 percent, etc. Likewise, though in the present embodiment the insert thickness is approximately 0.020 inch, other insert thicknesses could be used. Further, as mentioned the shape, size, and design of the insert can vary from what is shown in the accompanying drawings. The bismuth trioxide in one embodiment is added to the acetyl resin in powder form to define the mixture before molding, though other forms of bismuth trioxide or other suitable radiopaque material can also be employed.

The insert 750 is formed in one embodiment by injection molding, though in other embodiments other processes, including machining and other molding procedures, may be used. For instance, in one embodiment, the insert is formed by first extruding a length of extruded material, then slicing the extrusion into individual inserts. In another embodiment, the insert is provided by stamping or cutting the insert from a formed sheet of material including the base and radiopaque materials. These and other procedures are therefore contemplated.

Once formed, the insert 750 can be included in the access port 510 during manufacture of the access port. In one embodiment, inclusion of the insert 750 in the access port 510 is achieved via an insert-molding process, wherein the already-formed insert is placed into the access port mold, then the access port or a portion thereof is injection molded about the insert to ultimately produce a port appearing similar to that shown in FIG. 77, with the insert positioned substantially flush with the bottom surface 752 of the access port 510. Note that in one embodiment, a top or cap portion and a base portion of the access port are formed via separate molding processes. In this case, the insert is insert-molded into the base portion during molding thereof. Then, the cap and base portions of the access port are joined together via a suitable process, such as ultrasonic welding for instance. Energy transferred during the ultrasonic welding of the cap and base portions assists in solidifying the insert-molded bond between the insert and the base portion of the access port, in one embodiment.

Note that in other embodiments other processes can be used to mate the insert to the access port, including placement of the insert in a pre-defined recess of the access port, for instance. In the latter case, the insert could be ultrasonically welded into place within the recess, or by some other suitable attachment process.

Note that the access port 510 shown here includes both a cap 514 and the base 516, though in other embodiments, single piece or other types of multi-part ports can benefit from the principles described herein.

With the insert 750 positioned as shown in FIG. 77 so as to be visible from the port exterior, a clinician can view the identification feature 200 of the insert and ascertain the predetermined attribute of the port before implantation. After implantation, as mentioned, the insert 750 enables identification of the port attribute via observation of the identification feature 200 in a radiographic image of the access port 510.

Note that, because bismuth trioxide is not a metal, but rather a metal oxide, a non-metallic access port including an insert partially formed from bismuth trioxide can be used without difficulty in situations where the presence of metal is problematic, such as in magnetic resonance imaging (M.R.I.). Further, in the present embodiment the base material of the insert (acetyl resin) is substantially similar to the material from which the access port body is manufactured (also acetyl resin). As such, both include similar coefficients of expansion and contraction. This prevents warping of the insert as the insert and surrounding port body material cool after the insert molding process is complete. Also, because the insert includes a relatively soft base material, the mold will not be damaged if the insert is somehow malpositioned during the insertion molding process.

As mentioned, other materials can be employed in manufacturing the radiopaque insert 750 and other inserts described herein, including a suitable biocompatible base material in place of the acetyl resin and a suitable biocompatible radiopaque material in place of the bismuth trioxide. One suitable combination for forming the insert includes a base material of polycarbonate sold under the name MAKROLON^{®} 2558 and tungsten as the radiopaque material. Other suitable base materials include biocompatible thermoplastic materials. Other possible radiopaque materials include precious metals including gold, silver, etc., barium sulfate and other suitable sulfates, suitable oxides, and suitably dense ceramics including alumina, zirconia, etc. Such materials are therefore contemplated.

In one embodiment, it is appreciated that the use of a base material that is the same material employed for forming the access port body enables the insert to shrink at a similar rate to that of the port body during the molding process, thus desirably preventing warping of the of the port body or insert.

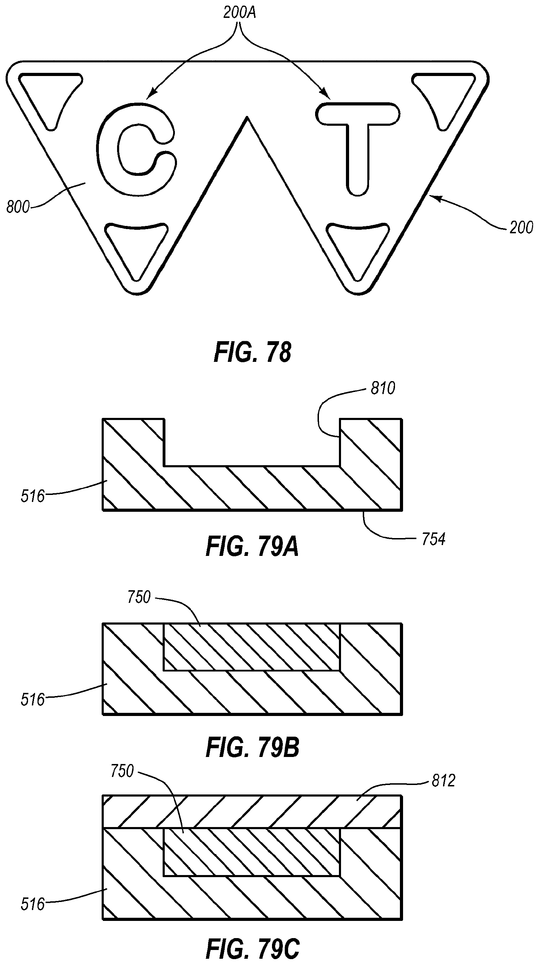

As mentioned, the insert including the identification feature can include other configurations, one example of which is shown in FIG. 78, wherein an insert 800 is shown for use in a double reservoir access port, such as one similar to that shown in FIGS. 66D and 67, for instance. As before, the insert 800 includes the identification feature 200, which in turn includes the alphanumeric indicia 200A. The shape of the insert 800 includes a connected triangle design, with each triangle including one of the two alphanumeric indicia 200A of "C" and "T" letter-shaped holes and triangular holes disposed at several of the vertices of the triangles.

Also as before, the composition of the insert 800 includes a mixture of acetyl resin and bismuth trioxide in relative concentrations similar to those of the previous embodiment so as to render the insert radiopaque when included in an access port or other implantable device and is radiographically imaged using x-ray imaging technology. Again, many different character, pattern, and/or combination configurations are possible. For instance, in addition to identifying the access port as power injectable, this and other identification features described herein can be used to designate lot numbers, hospital identification, port brand, etc.

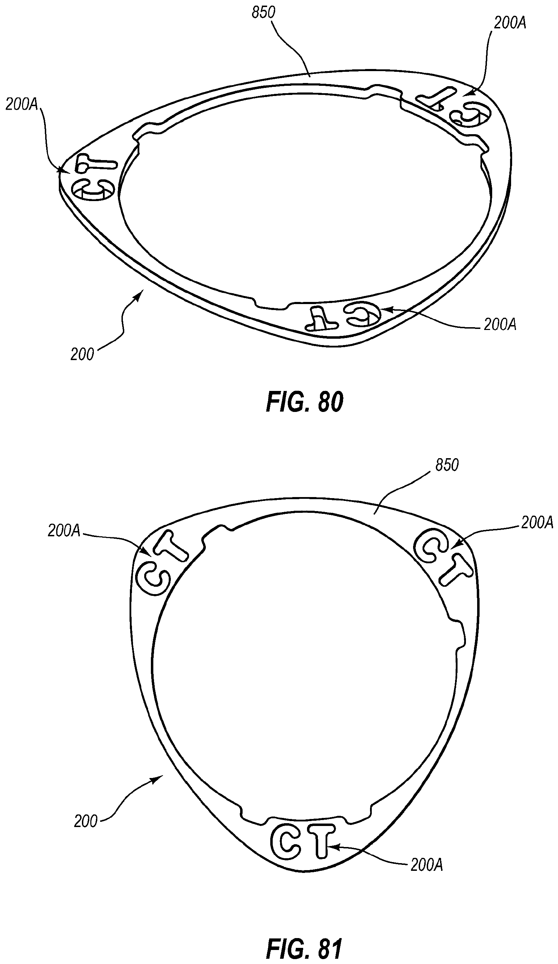

FIGS. 80 and 81 depict yet another possible configuration for an insert including the identification feature, wherein a component 850 is shown. The component 850 includes the identification feature 200, which in turn includes the alphanumeric indicia 200A for providing a radiographic confirmation of an aspect of the port or medical device with which the component 850 is included. In particular, the identification feature 200 of the component 850 includes three alphanumeric indicia 200A of "C" and "T" letter-shaped holes disposed at the vertices of the generally triangularly shaped component. In the present embodiment, the component 850 defines a hole for enabling the component to fit about an outer perimeter of an access port, though it is appreciated that other shapes and configurations are possible. As before, the composition of the component 850 in the present embodiment includes a mixture of acetyl resin and bismuth trioxide in relative concentrations similar to those of previous embodiments so as to render the component radiopaque when included with an access port or other implantable device and is radiographically imaged using x-ray imaging technology.

FIGS. 79A-79C depict one possible embodiment for placement of the insert 750 within the access port base 516 or other suitable portion of the access port, wherein a recess 810 is defined in a first molded portion of the port base. As shown in FIG. 79B, the radiopaque insert 750 - after formation thereof by a suitable process as described above - is placed in the recess 810, and an additional base portion 812 is formed over the recess by welding, overmolding or other suitable process. The insert 750 is thus encapsulated in the port base 516. Encapsulation of the insert in this manner can eliminate the need for use of biocompatible materials in the radiopaque insert. Note that the size and placement of both the recess and the insert within the access port can vary from what is shown here. For instance, the recess can include a slot on a portion of the port body that is sized to enable the insert to be slid therein, after which the slot is capped to cover the insert.

It is appreciated that a radiopaque identification feature in accordance with the principles described herein can be employed in other applications. For instance, in one embodiment, a radiopaque identification feature including a suitable base material and bismuth trioxide or other suitable radiopaque material described herein can be employed as a distal end plug for a lumen of a catheter. These and other possible applications are therefore contemplated.

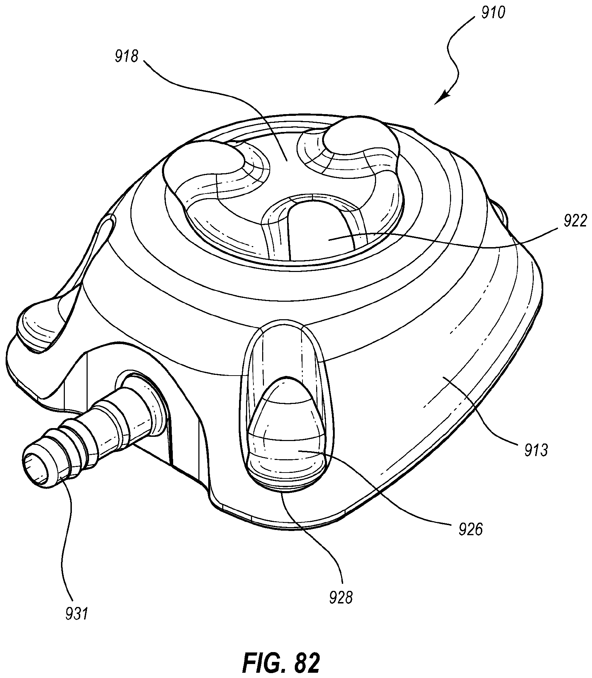

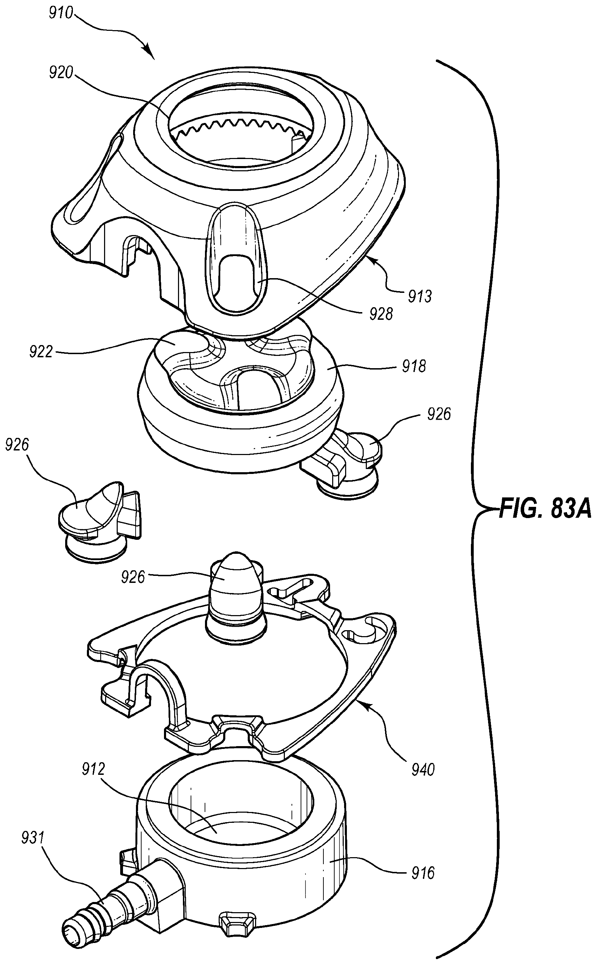

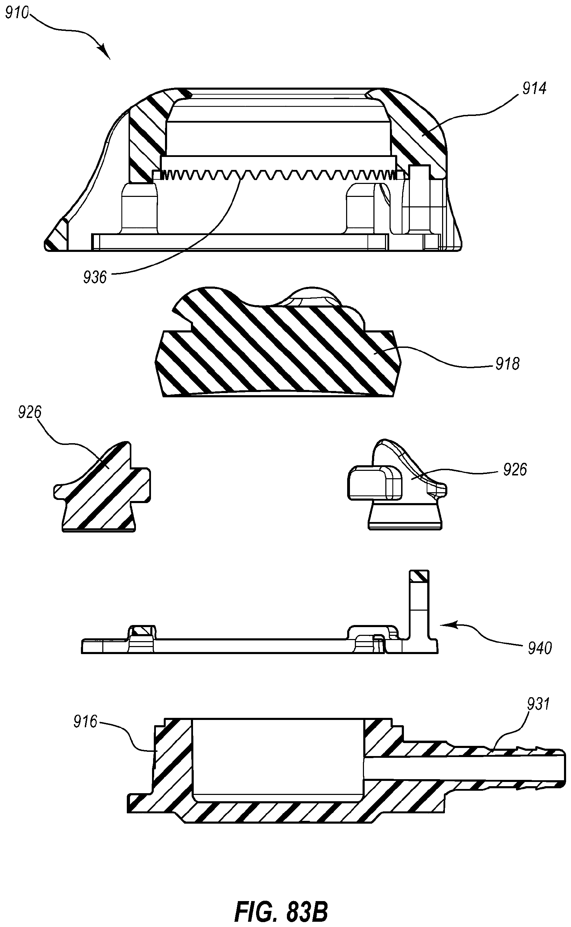

Reference is now made to FIGS. 82, 83A, and 83B in describing various details regarding an implantable access port ("port"), generally designated at 910, according to one embodiment. As shown, the port 910 includes a body 913 further including a base portion 916 and a cap portion 914 that are matable together to form the body. When mated together as seen in FIG. 82, the base and cap portions 916, 914 cooperate to define a fluid cavity 912, more clearly seen in FIG. 83A. The cap portion 914 defines an opening 920 into which is inserted a needle-penetrable septum 918 to provide needle access to the fluid cavity 912. The septum 918 optionally includes one or more palpation features 922 that enable the septum to be palpated and located under the skin by a clinician after implantation of the port. Note that the particular size, shape, and configuration of the port body and septum can vary from what is shown and described herein.

A stem 931 that provides a conduit in fluid communication with the fluid cavity 912 is integrally formed with the base portion 916 of the port body 913. Also, a plurality of suture plugs 926 is included, each plug being disposed in a corresponding suture hole 928 defined in the port body 913.

In the present embodiment, the port body 913 includes poly ether ether ketone ("PEEK"). PEEK is a relatively strong thermoplastic that enables the port 910 to withstand the pressures associated with power injection of viscous contrast media fluids through the port without rupture. Power injection flow rates are typically about 5 ml per second. The relative strength of PEEK also facilitates the integral formation of the stem 931 with the base portion 916, as best seen in FIGS. 83A and 83B, thus simplifying port body structure. Indeed, the relatively strong PEEK material allows the stem 931 to be integrally formed so as to define a sufficiently large inner diameter that enables power injection of fluids therethrough while maintaining sufficient stem strength to avoid fracture during use. Further, formation of the base portion 916 from a relatively strong material such as PEEK allows the bottom floor of the base defining the fluid cavity 912 to be relatively thin while still successfully resisting puncture thereof by a needle inserted into the port 910. Note that other suitable, relatively high-strength plastics may also be employed in other embodiments. The base portion 916 and cap portion 914 are formed via a suitable process, including injection molding, for instance.

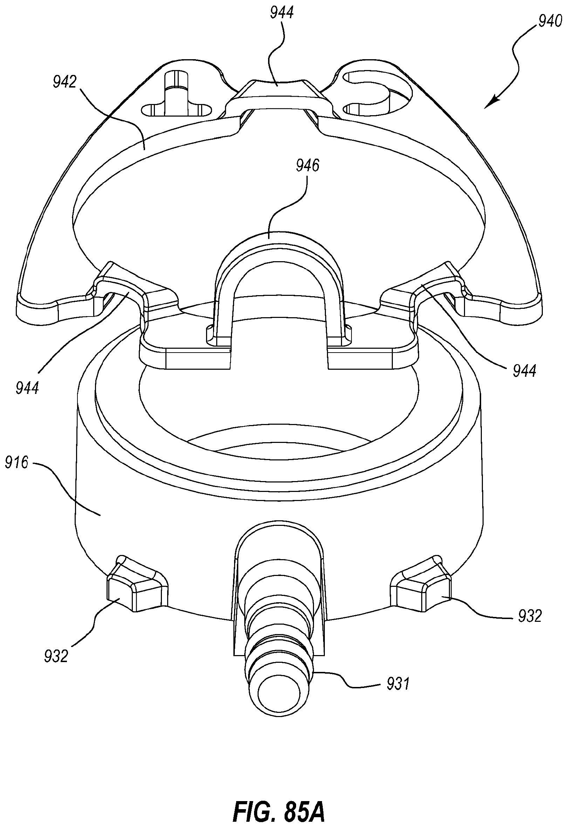

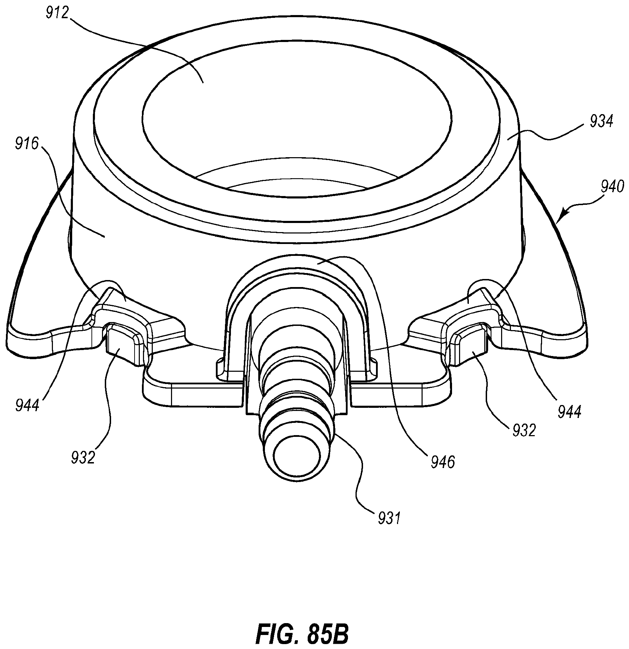

FIGS. 83A, 83B, 85A, and 85B show that the base portion 916 of the port body 913 includes a plurality of feet 932 that extend from the bottom portion of the base. The base portion 916 further defines a lip 934 on a top surface thereof that is employed in permanently mating the base portion with the cap portion 914 of the port body 913, as will be seen. The cap portion 914 includes a plurality of welding features 936 (FIG. 83B) circumferentially arranged about the opening 920 that are employed in mating the cap portion to the base portion 916, as will be seen. In the present embodiment, the welding features 936 are arranged as a plurality of triangular teeth, though in other embodiments other suitable welding features can be employed. The use of a plurality of discrete welding features 936 in the present embodiment provides for a relatively quicker completion of the ultrasonic welding procedure when compared to a singular welding feature configuration, such as an annular ridge.

As in previous embodiments discussed further above, FIGS. 83A and 83B show that the port 910 of the present embodiment includes the radiopaque identification feature 200 that is observable through interaction with an imaging technology, such as x-ray and fluoroscopy, for instance, in order to facilitate identification of at least one attribute or characteristic of an access port or other implantable medical device including the identification feature, subsequent to implantation of the device within the body of a patient. Again, it is appreciated that the embodiments to be described can be included alone or together with other identification features described herein and may be employed with access ports having a variety of sizes, shapes, and other variations in configuration. As such, the embodiments described herein are merely examples of the principles of the present disclosure.

The identification feature 200 in the present embodiment is implemented as a radiopaque insert 940. The insert 940 includes a sufficiently radiopaque material and a base material. In the present embodiment, bismuth trioxide is employed as the radiopaque material, intermixed with a base material of acetyl resin such as that commonly sold under the name DELRIN^{®}. In the present embodiment, the material composition for the radiopaque insert 940 includes about 30 percent bismuth trioxide and about 70 percent acetyl resin by weight, though these percentages can vary according to need or other factors. The insert 940 can be manufactured by various processes including molding, extrusion, machining, etc. In one embodiment, the insert 940 is manufactured to have a minimum thickness of about 0.033 inch, a length of about 0.8 inch and a width of about 0.6 inch, those these dimensions can vary according to application.

It is appreciated that other non-metallic radiopaque materials can be employed in the radiopaque insert in place of bismuth trioxide. Such materials include, as non-limiting examples, bismuth compounds including bismuth subcarbonate or bismuth oxychloride, ceramic powders and compounds including zirconium oxide, aluminum oxide, or titanium dioxide, etc. It is noted that bismuth trioxide, bismuth subcarbonate, bismuth oxychloride and other suitable compounds are relatively more radiopaque than barium sulfate, a typical radiopacifier, which enables relatively less radiopaque material to be needed in the radiopaque insert, thus altering to a lesser degree the properties of the acetyl resin or other base material of the radiopaque insert. In one embodiment, the insert can include from about 25 to about 40 percent bismuth oxychloride or bismuth subcarbonate by weight, with the balance being acetyl resin, though many other composition ratios are possible and contemplated herein. In another embodiment, the insert can include from about 45 to about 50 percent zirconium oxide by weight, with the balance being acetyl resin, though many other composition ratios are possible and contemplated herein. Also, note that other base materials can be employed in the radiopaque insert in place of acetyl resin.

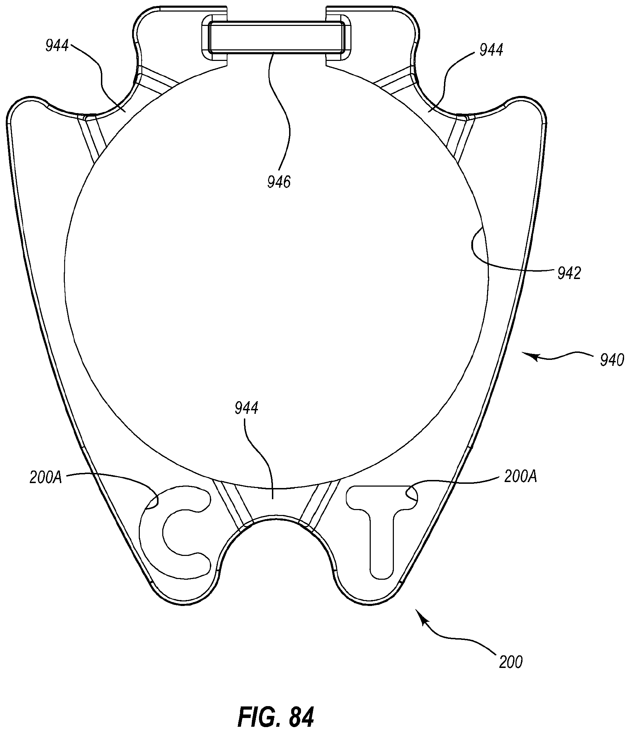

As shown in FIG. 84, the radiopaque insert 940 includes a body that is formed via molding, machining, extrusion, or other suitable mode of fabrication, as already mentioned. The body of the radiopaque insert 940 in the present embodiment roughly defines a triangular configuration and bears resemblance to an arrowhead shape. A central hole 942 is defined by the body of the radiopaque insert 940 to enable the insert to fit over the base portion 916 of the port body 913 (FIGS. 85A, 85B). Feet contours 944 and a stem contour 946 are also defined by the body of the radiopaque insert 940 to facilitate sandwiching of the radiopaque insert between the portions of the port body, as is described below. When viewed from a side configuration (FIG. 83B), the feet and stem contours 944 and 946 define raised segments in the body of the radiopaque insert 940. Of course, these raised features can vary in configuration to match the particular configuration of the port body.

The radiopaque insert 940 further includes one or more radiographically discernible identification features, or indicia, that assist in identifying the predetermined characteristic or attribute of the port 910. In the present embodiment, the radiopaque insert 940 includes alphanumeric indicia 200A, here implemented as the letters "C" and "T," as in embodiments discussed further above, which are generally recognized to indicate that the port 10 is capable of power injection at flow rates of, in one example, about 5 ml per second. Note that a variety of symbols, numbers, letters, or other indicia can be included on the radiopaque insert to convey to a clinician the predetermined characteristic of the port. Note that in the present embodiment, the predetermined characteristic is that of the power injectability of the port, though in other embodiments other characteristics, attributes, and/or properties can be conveyed by the indicia of the radiopaque insert.

FIGS. 85A and 85B show the manner of engagement of the radiopaque insert 940 with the port base 916 of the port body 913. As shown, the radiopaque insert 940 engages the base portion 916 such that the central hole 942 of the radiopaque insert receives the cylindrical body of the base portion. The radiopaque insert 940 seats on the base portion 916 such that the feet contours 944 each rest atop the corresponding feet 932 of the base portion and such that the stem contour 946 rests atop the stem 931, as shown in FIG. 85B.

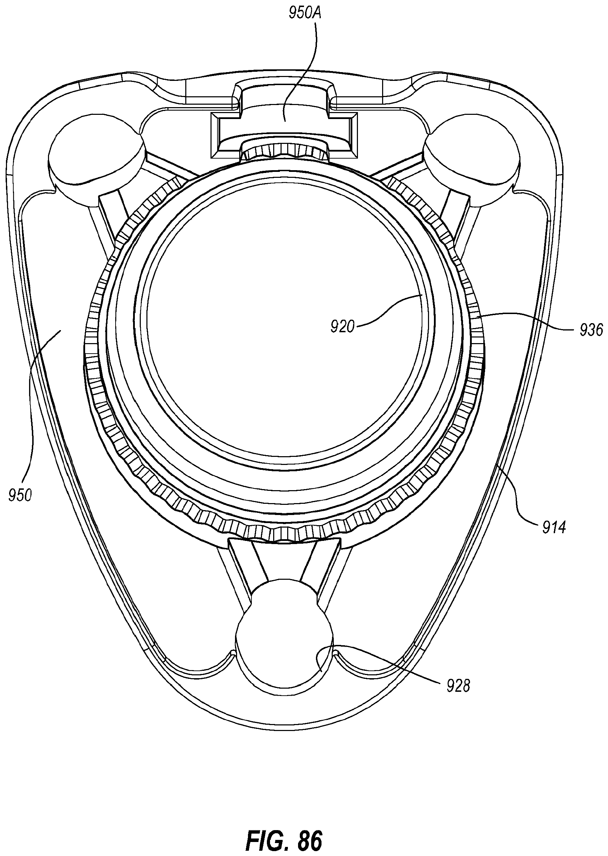

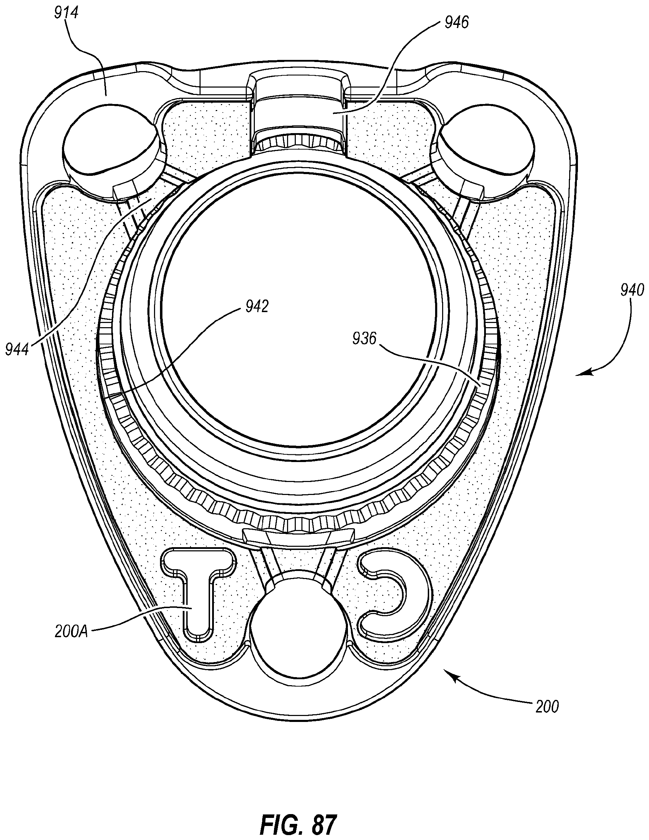

Similarly, FIGS. 86 and 87 show the manner engagement of the radiopaque insert 940 with the cap portion 914 of the port body 913. In detail, FIG. 86 shows that the cap portion 914 defines a recess 950 shaped to accommodate the radiopaque insert 940, including a stem recess 950A sized to accommodate the stem contour 946 of the radiopaque insert. Thus, when the radiopaque insert 940 is sandwiched between the cap portion 914 and the base portion 916, the feet contours 944 and stem contours 946 are interposed between correspondingly shaped features of the cap portion and base portion and or other parts of the port, in one embodiment.

Engagement of the radiopaque insert 940 with the cap 914 and base 916 as described above enables the insert to be interposed between the cap and base in a sandwiched configuration during manufacture of the port 910. Particularly, during port manufacture the radiopaque insert 940 is interposed between the port body base portion 916 and the cap portion 914 in a manner similar to that shown in FIGS. 85B or 87. Any suitable method can be employed to maintain the radiopaque insert in place, including positioning such that gravity maintains it in place, temporary fixation, etc.

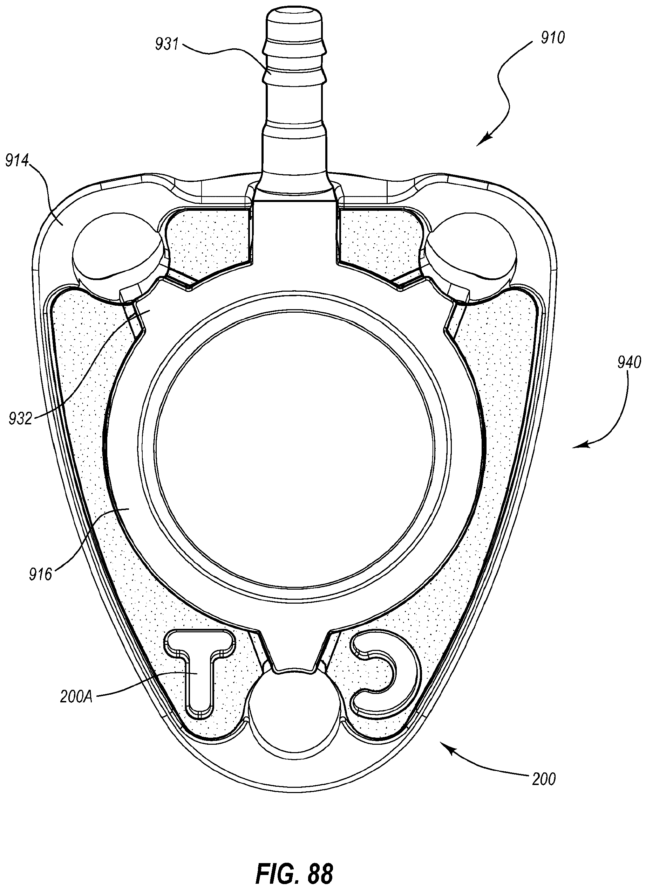

With the septum 918 (FIG. 83B) in place in the opening 920 of the cap portion 914, the base portion 916 and the cap portion are then mated together with the radiopaque insert 940 interposed therebetween. Ultrasonic welding is performed to bond the welding features 936 of the cap portion 914 to the lip 934 of the base portion 916. This sealably mates the base and cap portions 916, 914 together, which seals the fluid cavity 912 and secures the radiopaque insert 940 in a sandwiched configuration between the two port body portions, as shown in FIG. 88. In particular, note that the stem 931 and the feet 932 of the base 916 secure the insert 940 in place. Note also that the feet 932 of the base 916 secure each of the suture plugs 926 in place within the respective suture hole 928, in the present embodiment. As such, it is noted that the radiopaque insert is not permanently fastened in place by mechanical modes, but rather secured in place after welding is complete.

As shown in FIG. 88, in the present embodiment portions of the radiopaque insert 940 remain external to, and visible from the bottom of, the port 910 - including the ID feature 200 and its alphanumeric indicia 200A - so as to enable visual inspection and identification of the port prior to implantation. Correspondingly, the radiopacity of the insert 940 enables identification of the port 910 after implantation via x-ray imaging technology.

It is noted that in one embodiment, the base and cap portions can be welded together without the radiopaque insert included in the port without affecting the mating or integrity of the resultant access port. Note also that the principles described herein can be extended to a variety of medical devices and access ports, such as a dual reservoir access port, for instance.

Note that the inclusion of the radiopaque insert including acetyl resin and bismuth trioxide, together with formation of the port body from PEEK, produces a non- metallic port that is still identifiable via x-ray imaging technology. This in turn reduces or eliminates the risk of the port producing undesirable image artifacts when the patient undergoes an MRI, CT, or x-ray scan with the port subcutaneously in place within the patient's body. This further enables clearer MRI scans to be made, leading to more accurate diagnoses. Moreover and as mentioned, the present access port is configured in one embodiment to withstand power injection therethrough of contrast media or other viscous fluids. Such power injection is often performed in connection with CT scans, MRI scans, etc. in order to more accurately diagnose patient condition.

While certain representative embodiments and details have been shown for purposes of illustrating aspects contemplated by the instant disclosure, it will be apparent to those skilled in the art that various changes in the methods and apparatus disclosed herein may be made without departing from the scope contemplated by the instant disclosure, which is defined in the appended claims. For example, other access port sizes and shapes may be employed; and various other embodiments and structures may be employed for forming at least one identifiable feature of an access port contemplated by the instant disclosure. In particular, the access port may be formed in any number of shapes and sizes, such that any number of modifications and changes are possible to any of the embodiments described and illustrated herein without departing from the scope of the instant disclosure.

## Claims

1. A method for manufacturing a power injectable access port (910), comprising:
providing a port body (913) and a septum (918), the port body including a base portion (916) and a cap portion (914);
providing a radiopaque insert (940) including at least one indicia (200) observable via x-ray imaging technology subsequent to subcutaneous implantation of the power injectable access port (910) in a patient, the at least one indicia (200) conveying to an observer that the power injectable access port (910) is power injectable, the radiopaque insert (940) further comprising a central hole (942);
interposing the radiopaque insert (940) between the base portion (916) and the cap portion (914) such that a contoured portion (944, 946) of the radiopaque insert fits between correspondingly shaped features of the base portion (916) and the cap portion (914), and wherein the radiopaque insert (940) engages the base portion (916) such that the central hole (942) of the radiopaque insert (940) receives a cylindrical body of the base portion; and
cooperatively mating the base portion (916) to the cap portion (914) with the radiopaque insert (940) interposed therebetween such that a portion of the radiopaque insert (940) remains external to the port body (913).

2. The method for manufacturing according to claim 1, further comprising covering an opening (920) in the cap portion (914) with the septum (918), the opening (920) in communication with a fluid cavity (912) defined by the base portion (916) and the cap portion (914).

3. The method for manufacturing according to claim 2, wherein the base portion (916) includes a stem (931) integrally formed therewith, the stem (931) in fluid communication with the fluid cavity (912), optionally wherein the interposing includes fitting the contoured portion (944, 946) between the stem (931) and a corresponding recess (950A) defined by the cap portion (914).

4. The method for manufacturing according to claim 1, wherein the base portion (916) includes a plurality of extending feet (932), and wherein the radiopaque insert (940) comprises a plurality of contoured portions (944), the interposing comprising fitting each of the plurality of contoured portions (944) about each of the plurality of extending feet (932).

5. The method for manufacturing according to claim 1, wherein the radiopaque insert (940) includes a radiopaque material selected from the group consisting of bismuth trioxide, bismuth subcarbonate, bismuth oxychloride, zirconium oxide, aluminum oxide, titanium dioxide, and combinations thereof.

6. The method for manufacturing according to claim 1, wherein the radiopaque insert (940) is provided by injection molding a mixture including about 70 percent acetyl resin and about 30 percent bismuth trioxide by weight.

7. The method for manufacturing according to claim 1, wherein cooperatively mating the base portion (916) to the cap portion (914) further comprises ultrasonically welding the base portion (916) to the cap portion (914).

8. The method for manufacturing according to claim 1, wherein a plurality of ultrasonic welding features (936) are disposed on the cap portion and arranged in a circular pattern about a fluid cavity (912), each welding feature defining a triangular tooth, optionally wherein cooperatively mating the base portion (916) to the cap portion (914) further comprises ultrasonically welding the cap portion (914) to the base portion (916).

9. The method for manufacturing according to claim 1, wherein the radiopaque insert (940) is provided via extruding a length of material and slicing the length of material into the radiopaque insert (940).

10. The method for manufacturing according to claim 1, wherein the radiopaque insert (940) is provided via stamping the radiopaque insert (940) from a formed sheet of material including a base material and a radiopaque material.

## Patentansprüche

1. Verfahren zum Herstellen eines durch Kraft injizierbaren Zugangsanschlusses (910), umfassend:
Bereitstellen eines Anschlusskörpers (913) und eines Septums (918), wobei der Anschlusskörper einen Basisteil (916) und einen Kappenteil (914) einschließt;
Bereitstellen eines strahlungsundurchlässigen Einsatzes (940), einschließlich mindestens einer Markierung (200), das mittels Röntgenbildgebungstechnologie nach einer subkutanen Implantation des durch Kraft injizierbaren Zugangsanschlusses (910) in einen Patienten beobachtbar ist, wobei die mindestens eine Markierung (200) einem Beobachter übermittelt, dass der durch Kraft injizierbare Zugangsanschluss (910) durch Kraft injizierbar ist, wobei der strahlungsundurchlässige Einsatz (940) weiter ein zentrales Loch (942) umfasst;
Einfügen des strahlungsundurchlässigen Einsatzes (940) zwischen den Basisteil (916) und den Kappenteil (914), so dass ein konturierter Abschnitt (944, 946) des strahlungsundurchlässigen Einsatzes zwischen entsprechend geformten Merkmalen des Basisteils (916) und des Kappenteils (914) passt, und wobei der strahlungsundurchlässige Einsatz (940) in den Basisteil (916) eingreift, so dass das zentrale Loch (942) des strahlungsundurchlässigen Einsatzes (940) einen zylindrischen Körper des Basisteils aufnimmt; und
Zusammenwirken des Basisteils (916) mit dem Kappenteil (914), wobei der strahlungsundurchlässige Einsatz (940) dazwischen eingefügt ist, so dass ein Abschnitt des strahlungsundurchlässigen Einsatzes (940) außerhalb des Anschlusskörpers (913) verbleibt.

2. Verfahren zum Herstellen nach Anspruch 1, weiter umfassend Abdecken einer Öffnung (920) im Kappenteil (914) mit dem Septum (918), wobei die Öffnung (920) mit einem Fluidhohlraum (912) in Verbindung steht, der durch den Basisteil (916) und den Kappenteil (914) definiert ist.

3. Verfahren zum Herstellen nach Anspruch 2, wobei der Basisteil (916) einen integral damit ausgebildeten Schaft (931) einschließt, wobei der Schaft (931) mit dem Fluidhohlraum (912) in Fluidverbindung steht, wobei optional das Einfügen ein Einpassen des konturierten Abschnitts (944, 946) zwischen den Schaft (931) und eine entsprechende Aussparung (950A) einschließt, die durch den Kappenteil (914) definiert ist.

4. Verfahren zum Herstellen nach Anspruch 1, wobei der Basisteil (916) eine Vielzahl von sich erstreckenden Füßen (932) einschließt, und wobei der strahlungsundurchlässige Einsatz (940) eine Vielzahl von konturierten Abschnitten (944) umfasst, wobei das Einfügen ein Einpassen jedes der Vielzahl von konturierten Abschnitten (944) um jeden der Vielzahl von sich erstreckenden Füßen (932) umfasst.

5. Verfahren zum Herstellen nach Anspruch 1, wobei der strahlungsundurchlässige Einsatz (940) ein strahlungsundurchlässiges Material einschließt, das aus der Gruppe ausgewählt ist, bestehend aus Bismuttrioxid, Bismutsubcarbonat, Bismutoxychlorid, Zirkoniumoxid, Aluminiumoxid, Titandioxid und Kombinationen davon.

6. Verfahren zum Herstellen nach Anspruch 1, wobei der strahlungsundurchlässige Einsatz (940) durch Spritzgießen einer Mischung bereitgestellt wird, die etwa 70 Gewichtsprozent Acetylharz und etwa 30 Gewichtsprozent Bismuttrioxid einschließt.

7. Verfahren zum Herstellen nach Anspruch 1, wobei das Zusammenwirken des Basisteils (916) mit dem Kappenteil (914) weiter ein Ultraschallschweißen des Basisteils (916) mit dem Kappenteil (914) umfasst.

8. Verfahren zum Herstellen nach Anspruch 1, wobei eine Vielzahl von Ultraschallschweißmerkmalen (936) auf dem Kappenteil angeordnet sind und in einem kreisförmigen Muster um einen Fluidhohlraum (912) herum angeordnet sind, wobei jedes Schweißmerkmal einen dreieckigen Zahn definiert, wobei optional das Zusammenwirken des Basisteils (916) mit dem Kappenteil (914) weiter ein Ultraschallschweißen des Kappenteils (914) mit dem Basisteil (916) umfasst.

9. Verfahren zum Herstellen nach Anspruch 1, wobei der strahlungsundurchlässige Einsatz (940) durch Extrudieren einer Materiallänge und Schneiden der Materiallänge zu dem strahlungsundurchlässigen Einsatz (940) bereitgestellt wird.

10. Verfahren zum Herstellen nach Anspruch 1, wobei der strahlungsundurchlässige Einsatz (940) durch Ausstanzen des strahlungsundurchlässigen Einsatzes (940) aus einem geformten Materialblatt bereitgestellt wird, das ein Basismaterial und ein strahlungsundurchlässiges Material einschließt.

## Revendications

1. Procédé de fabrication d'un port d'accès à injection automatique (910), comprenant :
la fourniture d'un corps (913) de port et d'un septum (918), le corps de port incluant une partie base (916) et une partie coiffe (914) ;
la fourniture d'un insert radioopaque (940) incluant au moins un indice (200) observable via une technologie d'imagerie à rayons x suite à l'implantation sous-cutanée du port d'accès à injection automatique (910) dans un patient, le au moins un indice (200) communiquant à un observateur que le port d'accès à injection automatique (910) est injection automatique, l'insert radioopaque (940) comprenant en outre un trou central (942) ;
l'interposition de l'insert radioopaque (940) entre la partie base (916) et la partie coiffe (914) de telle sorte qu'une partie profilée (944, 946) de l'insert radioopaque s'ajuste entre des caractéristiques de forme correspondante de la partie base (916) et de la partie coiffe (914), et dans lequel l'insert radioopaque (940) met en prise la partie base (916) de telle sorte que le trou central (942) de l'insert radioopaque (940) reçoit un corps cylindrique de la partie base ; et
l'appariement coopératif de la partie base (916) sur la partie coiffe (914) avec l'insert radioopaque (940) interposé entre elles de telle sorte qu'une partie de l'insert radioopaque (940) reste extérieur au corps (913) de port.

2. Procédé de fabrication selon la revendication 1, comprenant en outre la couverture d'une ouverte (920) dans la partie coiffe (914) avec le septum (918), l'ouverture (920) étant en communication avec une cavité (912) de fluide définie par la partie base (916) et la partie coiffe (914).

3. Procédé de fabrication selon la revendication 2, dans lequel la partie base (916) inclut une tige (931) formée d'un seul tenant avec celle-ci, la tige (931) étant en communication fluidique avec la cavité (912) de fluide, facultativement dans lequel l'interposition inclut l'ajustement de la partie profilée (944, 946) entre la tige (931) et un évidement (950A) correspondant défini par la partie coiffe (914).

4. Procédé de fabrication selon la revendication 1, dans lequel la partie base (916) inclut une pluralité de pieds extensibles (932), et dans lequel l'insert radioopaque (940) comprend une pluralité de parties profilées (944), l'interposition comprenant l'ajustement de chacune de la pluralité de parties profilées (944) autour de chacun de la pluralité de pieds extensibles (932).

5. Procédé de fabrication selon la revendication 1, dans lequel l'insert radioopaque (940) inclut un matériau radioopaque choisi à partir du groupe consistant en trioxyde de bismuth, sous-carbonate de bismuth, oxychlorure de bismuth, oxyde de zirconium, oxyde d'aluminium, dioxyde de titane, et des combinaisons de ceux-ci.

6. Procédé de fabrication selon la revendication 1, dans lequel l'insert radioopaque (940) est fourni par moulage par injection d'un mélange incluant environ 70 pour cent de résine d'acétyle et environ 30 pour cent de trioxyde de bismuth en poids.

7. Procédé de fabrication selon la revendication 1, dans lequel l'appariement coopératif de la partie base (916) sur la partie coiffe (914) comprend en outre le soudage par ultrasons de la partie base (916) sur la partie coiffe (914).

8. Procédé de fabrication selon la revendication 1, dans lequel une pluralité de caractéristiques (936) de soudage par ultrasons sont disposés sur la partie coiffe et agencés selon un motif circulaire autour d'une cavité (912) de fluide, chaque caractéristique de soudage définissant une dent triangulaire, facultativement dans lequel l'appariement coopératif de la partie base (916) sur la partie coiffe (914) comprend en outre le soudage par ultrasons de la partie coiffe (914) sur la partie base (916).

9. Procédé de fabrication selon la revendication 1, dans lequel l'insert radioopaque (940) est fourni via l'extrusion d'une longueur de matériau et le tranchage de la longueur de matériau dans l'insert radioopaque (940).

10. Procédé de fabrication selon la revendication 1, dans lequel l'insert radioopaque (940) est fourni par l'estampage de l'insert radioopaque (940) depuis une feuille formée de matériau incluant un matériau de base et un matériau radioopaque.
